# FASCICULE DE BREVET EUROPEEN

(11) **EP 4 220 141 B1**
(45) Date de publication et mention de la délivrance du brevet: **26.03.2025**
(21) Numéro de dépôt: 22184795.7
(22) Date de dépôt: 30.01.2018
(51) Int. Cl.: G01N 21/85

(54) **PROCÉDÉ ET DISPOSITIF D'ANALYSE OPTIQUE DE FRUITS OU LÉGUMES ET DISPOSITIF DE TRI AUTOMATIQUE**
VERFAHREN UND VORRICHTUNG ZUR OPTISCHEN ANALYSE VON OBST ODER GEMÜSE UND AUTOMATISCHE SORTIERVORRICHTUNG
METHOD AND DEVICE FOR OPTICAL ANALYSIS OF FRUIT AND VEGETABLES AND AUTOMATIC SORTING DEVICE

(30) Priorité: 01.03.2017 FR 1751683
(43) Date de publication de la demande: 02.08.2023
(62) Demande divisionnaire de: 18705439.0
(73) Titulaire: MAF Agrobotic, 82000 Montauban (FR)
(72) Inventeur: BLANC, Philippe, 82000 MONTAUBAN (FR)
(74) Mandataire: BARRE LAFORGUE

(56) Documents cités:
- WO-A1-2016/018157
- WO-A2-2016/054408
- FR-A1- 2 874 424
- FR-A1- 2 985 025
- US-A1- 2002 008 055
- MAYANK GOEL ET AL: "HyperCam", PERVASIVE AND UBIQUITOUS COMPUTING, ACM, 2 PENN PLAZA, SUITE 701 NEW YORK NY 10121-0701 USA, 7 September 2015 (2015-09-07), pages 145 - 156, XP058073983, ISBN: 978-1-4503-3574-4, DOI: 10.1145/2750858.2804282
- SUN JASON ET AL: "Multispectral scattering imaging and NIR interactance for apple firmness predictions", POSTHARVEST BIOLOGY AND TECHNOLOGY, vol. 119, 2016, pages 58 - 68, XP029572710, ISSN: 0925-5214, DOI: 10.1016/J.POSTHARVBIO.2016.04.019
- N. ALEIXOS ET AL: "Multispectral inspection of citrus in real-time using machine vision and digital signal processors", COMPUTERS AND ELECTRONICS IN AGRICULTURE, vol. 33, no. 2, 1 February 2002 (2002-02-01), AMSTERDAM, NL, pages 121 - 137, XP055234429, ISSN: 0168-1699, DOI: 10.1016/S0168-1699(02)00002-9

## Description

L'invention concerne un procédé et un dispositif d'analyse optique de fruits ou légumes en vue de leur tri automatique. Elle s'étend à un dispositif de tri automatique de fruits ou légumes mettant en œuvre un procédé d'analyse optique selon l'invention et/ou comportant un dispositif d'analyse optique selon l'invention.

On sait que l'analyse optique des fruits ou légumes par imagerie dans une bande de longueurs d'onde comprise typiquement entre 250 nm et 1000 nm, c'est-à-dire des ultraviolets jusqu'aux infrarouges en passant par le domaine visible, permet de mesurer sans contact différents paramètres, notamment les dimensions (calibre), le taux de sucre, l'acidité, le degré de maturité, la fermeté, la présence de défauts externes ou internes, la couleur... Pour ce faire cependant, il est nécessaire de multiplier les dispositifs d'éclairage et/ou les dispositifs de prise de vues pour activer, pour chaque paramètre à mesurer, une analyse optique dans un domaine de longueur d'onde approprié à la mesure de ce paramètre. En outre, la mesure d'un même paramètre peut nécessiter plusieurs domaines de longueur d'onde d'éclairage et/ou plusieurs domaines de longueur d'onde de prise de vues, voire plusieurs images différentes pour un même domaine de longueur d'onde d'éclairage et/ou de prise de vues, par exemple une image en réflexion et une image en transmission.

La publication Sun Jason et al « Multispectral scattering imaging and NIR interactance for apple firmess predictions » Potharvest Biology and Technology, vol.119, 2016, pages 58-68 décrit une technique d'imagerie multispectrale de mesures de spectroscopie par réflectance à résolution spatiale permettant d'évaluer la fermeté de pommes. Cette technique consiste à appliquer sur la pomme des faisceaux laser à différentes longueurs d'onde focalisés par une ouverture de 50 µm, et à capter l'image rétrodiffusée par la pomme. Outre qu'elle est limitée à la prédiction de la fermeté, cette technique ne permet pas d'analyser tous les défauts possibles sur toute la surface de la pomme.

Il est à noter que les techniques d'analyse optique par imagerie se distinguent en particulier des techniques spectrométriques ou spectroscopiques qui ne permettent que l'analyse de la lumière en provenance d'une source focalisée telle qu'une fibre optique ou un laser, et donc d'une portion ponctuelle très localisée de chaque objet.

Par ailleurs, l'analyse optique des objets doit être effectuée sur les différentes portions de surface extérieure des objets, qui sont en général entraînés en rotation alors qu'ils sont transportés en regard des dispositifs d'éclairage et des caméras (cf. par exemple WO 01/01071, FR 2874424). Il n'est pas rare qu'une analyse optique dans un seul domaine de longueur d'onde d'éclairage et/ou de prise de vues nécessite déjà une multiplicité de dispositifs d'éclairage et/ou de caméras.

Pour réaliser une analyse optique par imagerie dans plusieurs domaines de longueur d'onde, il est donc nécessaire de multiplier les dispositifs d'éclairage et/ou les caméras et/ou les filtres placés devant les dispositifs d'éclairage ou devant les caméras pour sélectionner les différents domaines de longueur d'onde. Pour des prises de vues dans le domaine des ultraviolets et des infrarouges, on utilise au moins une caméra monochrome. Pour des prises de vues dans le domaine visible, on utilise en général au moins une caméra couleur, à savoir une caméra trichromique comprenant au moins un capteur CMOS ou CCD ou autre.

Ainsi, en pratique, jusqu'à maintenant chaque poste d'analyse optique d'un dispositif de tri automatique de fruits ou légumes comporte une pluralité de caméras, au moins une caméra par domaine de longueur d'onde de prise de vues, c'est-à-dire classiquement entre quatre et huit caméras par ligne pour l'imagerie de chaque portion de surface extérieure de chaque objet.

Ces différents dispositifs sont particulièrement chers, fragiles, volumineux et encombrants, et nécessitent une maintenance régulière et coûteuse. Il est à noter à ce titre en particulier qu'ils sont soumis à des conditions environnementales relativement sévères (humides et sales), qui sont celles du traitement et du tri de fruits ou légumes.

En outre, l'utilisation de filtres pour sélectionner les longueurs d'onde est particulièrement coûteuse, de tels filtres étant onéreux à l'achat et se dégradant rapidement dans le temps.

Également, la transmission à un automate, formé d'un système informatique, des différentes images délivrées par les différentes caméras, et leur traitement par ce système informatique, notamment en vue du tri automatique des objets, nécessitent une durée qui, bien que faible en valeur absolue, n'est plus négligeable dans le contexte des dispositifs de tri autoniatique les plus modernes qui peuvent fonctionner à très haute cadence, typiquement à plus de dix -notamment jusqu'à environ cinquante, voire plus- fruits ou légumes par seconde et par ligne de convoyage. Ainsi, cette durée de transmission et de traitement dans les procédés et les dispositifs d'analyse optique connus est susceptible de constituer un frein à l'augmentation des cadences de certains dispositifs de tri automatique de fruits ou légumes.

L'invention vise donc à pallier ces inconvénients.

Elle vise en particulier à proposer un procédé et un dispositif d'analyse optique de fruits ou légumes par imagerie dans plusieurs domaines de longueur d'onde qui soient considérablement simplifiés, et moins coûteux à l'installation, à l'utilisation et à la maintenance.

Elle vise en particulier à proposer un tel procédé et un tel dispositif d'analyse optique pouvant être exempts de filtres pour la sélection des domaines de longueur d'onde de prise de vues.

Elle vise en particulier à proposer un tel procédé et un tel dispositif d'analyse optique qui soient mieux compatibles avec les conditions environnementales du tri automatique de fruits ou légumes, et permettent de mesurer différents paramètres à très haute cadence et à moindre coût. Elle vise en particulier à proposer une analyse optique de fruits ou légumes par imagerie qui soit compatible avec un tri automatique à très haute cadence, c'est-à-dire qui ne constitue pas en elle-même une limite à l'augmentation de la cadence du tri automatique.

Elle vise également à permettre une analyse optique de l'intégralité des critères de tri, autres que le poids, des fruits ou légumes exclusivement par imagerie dans différents domaines de longueur d'onde.

Elle vise également à proposer un dispositif de tri automatique d'objets tels que des fruits ou légumes présentant les mêmes avantages.

Dans tout le texte, on adopte la terminologie suivante :
- « rayonnement lumineux » désigne tout rayonnement électromagnétique de longueur d'onde comprise entre 200 nm et 1000 nm ;
- « domaine de longueur d'onde » désigne une longueur d'onde ou une bande de longueurs d'onde ;
- « domaine visible » désigne tout domaine de longueur d'onde inclus dans la bande des longueurs d'onde comprises entre 380 nm et 700 nm ;
- « domaine infrarouge » désigne tout domaine de longueur d'onde inclus dans la bande des longueurs d'onde comprises entre 700 nm et 1000 nm ;
- « domaine ultraviolet » désigne tout domaine de longueur d'onde inclus dans la bande des longueurs d'onde comprises entre 200 nm et 380 nm.

L'invention concerne donc un procédé d'analyse optique d'objets appartenant au groupe des fruits et légumes dans lequel des images représentatives des objets sont réalisées dans différents domaines de longueur d'onde de prise de vue, caractérisé en ce que :
- une pluralité de sources lumineuses formées de diodes électroluminescentes sont agencées pour pouvoir appliquer chacune un rayonnement lumineux sur au moins une portion de surface extérieure d'au moins un objet, dit objet éclairé, les différentes sources lumineuses étant adaptées pour pouvoir appliquer sélectivement sur chaque objet éclairé des rayonnements lumineux dans différents domaines de longueur d'onde d'éclairage,
- le rayonnement lumineux issu d'au moins une source lumineuse est appliqué sur l'intégralité d'une face visible (par ce rayonnement lumineux, c'est-à-dire, selon la direction de propagation de ce rayonnement lumineux) de la surface extérieure de chaque objet éclairé par cette source lumineuse,

- les sources lumineuses sont commandées selon une séquence d'éclairage prédéterminée de chaque objet éclairé, successivement selon lesdits différents domaines de longueur d'onde d'éclairage,
- des images sont réalisées par au moins une -notamment une et une seule-caméra couleur sensible aux rayonnements lumineux dans le domaine visible et aux rayonnements lumineux dans le domaine des infrarouges, dite caméra multispectrale, orientée vers une portion de surface extérieure d'au moins un objet éclairé correspondant à toute la face visible de la surface extérieure de l'objet selon l'axe optique de la caméra multispectrale et dont l'exposition est commandée en synchronisme avec ladite séquence d'éclairage de façon à réaliser avec cette même caméra multispectrale une pluralité d'images dans différents domaines de longueur d'onde de prise de vues de ladite portion de surface extérieure d'au moins un objet éclairé, dont au moins une image dans un domaine visible et au moins une image dans un domaine infrarouge,
- chaque caméra multispectrale est choisie dans le groupe des caméras comprenant un capteur CMOS avec une matrice de filtres de couleurs, exemptes de filtre coupant les infrarouges ; et des caméras comprenant trois capteurs CMOS, un capteur CMOS pour chaque couleur primaire, exemptes de filtre coupant les infrarouges.

L'invention s'étend également à un dispositif d'analyse optique d'objets appartenant au groupe des fruits et légumes comprenant des moyens pour réaliser des images des objets dans différents domaines de longueur d'onde, caractérisé en ce qu'il comporte :
- un dispositif d'éclairage comprenant une pluralité de sources lumineuses formées de diodes électroluminescentes agencées pour pouvoir appliquer chacune un rayonnement lumineux sur au moins une portion de surface extérieure d'au moins un objet, dit objet éclairé, les différentes sources lumineuses étant adaptées pour pouvoir appliquer sélectivement sur chaque objet éclairé des rayonnements lumineux dans différents domaines de longueur d'onde d'éclairage,
- le dispositif d'éclairage étant adapté pour pouvoir appliquer un rayonnement lumineux issu d'au moins une source lumineuse sur l'intégralité d'une face visible de la surface extérieure de chaque objet éclairé par cette source lumineuse,
- un dispositif de commande adapté pour pouvoir commander ces sources lumineuses selon une séquence d'éclairage prédéterminée de chaque objet éclairé, successivement selon lesdits différents domaines de longueur d'onde d'éclairage,
- au moins une -notamment une et une seule- caméra couleur sensible aux rayonnements lumineux dans le domaine visible et aux rayonnements lumineux dans le domaine des infrarouges, dite caméra multispectrale, orientée vers une portion de surface extérieure d'au moins un objet éclairé correspondant à toute la face visible de la surface extérieure de l'objet selon l'axe optique de la caméra multispectrale,
- chaque caméra multispectrale étant choisie dans le groupe des caméras comprenant un capteur CMOS avec une matrice de filtres de couleurs, exemptes de filtre coupant les infrarouges ; et des caméras comprenant trois capteurs CMOS, un capteur CMOS pour chaque couleur primaire, exemptes de filtre coupant les infrarouges,
et en ce que ledit dispositif de commande est adapté pour commander l'exposition de chaque caméra multispectrale en synchronisme avec ladite séquence d'éclairage de façon à réaliser avec cette même caméra multispectrale une pluralité d'images dans différents domaines de longueur d'onde de prise de vues de ladite portion de surface extérieure d'au moins un objet éclairé, dont au moins une image dans un domaine visible et au moins une image dans un domaine infrarouge.

L'invention s'étend également à un dispositif de tri automatique d'objets appartenant au groupe des fruits et légumes selon des critères de tri prédéterminés comprenant :
- au moins une ligne de convoyage apte à transporter les objets en regard de postes d'analyse des objets selon lesdits critères de tri, dont au moins un poste d'analyse optique,
- un automate relié aux postes d'analyse pour en recevoir des signaux d'analyse,
- des postes de déchargement des objets dans une pluralité de zones de déchargement,
- l'automate étant programmé pour commander le déchargement sélectif de chaque objet dans une zone de déchargement sélectionnée selon les signaux d'analyse reçus par cet automate pour cet objet,
caractérisé en ce qu'il comporte au moins un poste d'analyse optique formé d'un dispositif d'analyse optique selon l'invention.

L'inventeur a en effet constaté avec surprise qu'il est possible d'utiliser une même caméra couleur sensible aux rayonnements lumineux dans le domaine visible et aux rayonnements lumineux dans le domaine des infrarouges pour réaliser une pluralité d'images dans différents domaines de longueur d'onde de prise de vues, dont au moins une image dans un domaine visible et au moins une image dans un domaine infrarouge. Il suffit en effet d'une part de choisir une caméra couleur qui soit sensible aux infrarouges (et en particulier qui soit exempte de filtre coupant les infrarouges), et, si nécessaire, d'ajuster les réglages de cette caméra couleur pour la réalisation de chaque image dans un domaine infrarouge, en fonction de la sensibilité de chaque couleur de la caméra dans le domaine infrarouge considéré.

Grâce à l'invention, toutes les images nécessaires pour l'analyse optique des objets dans les domaines visibles et infrarouges peuvent être réalisées avec une seule et même caméra multispectrale commandée en synchronisme avec une séquence d'éclairage appropriée. Il en résulte une économie de moyens considérable et une grande simplification de chaque poste d'analyse optique.

Dans certains modes de réalisation avantageux d'un procédé selon l'invention, on utilise au moins une caméra multispectrale dotée d'une mémoire tampon d'enregistrement des différentes images pouvant être prises par cette caméra. Ainsi, ladite pluralité d'images de ladite portion de surface extérieure réalisée par une même caméra multispectrale est enregistrée dans une mémoire tampon de cette caméra multispectrale. De même, avantageusement, dans un dispositif d'analyse optique selon l'invention, chaque caméra multispectrale est dotée d'une mémoire tampon de mémorisation des images. Ainsi, les différentes images réalisées pendant chaque séquence d'éclairage peuvent être réalisées à très haute vitesse, à la vitesse maximale d'acquisition d'images par chaque caméra multispectrale) et mémorisées dans une mémoire tampon de chaque caméra multispectrale avant leur transmission à un automate (système informatique) de traitement de ces images, qui peut réaliser un traitement d'images à vitesse beaucoup plus lente. En conséquence, en particulier, la bande passante de la liaison entre chaque caméra multispectrale (qui est typiquement une liaison USB3) et l'automate ne constitue plus une limite à l'augmentation des cadences du tri automatique des fruits ou légumes.

Chaque caméra multispectrale est une caméra couleur, c'est-à-dire une caméra trichromique présentant des capteurs sensibles respectivement à l'une des trois couleurs primaires rouge, vert et bleu. Toute caméra couleur également sensible aux infrarouges, c'est-à-dire notamment exempte de filtre coupant les infrarouges, peut être utilisée à titre de caméra multispectrale dans un dispositif et un procédé d'analyse optique selon l'invention. En particulier, chaque caméra multispectrale peut être choisie dans le groupe des caméras comprenant un capteur CMOS (avec une matrice de filtres de couleurs telle qu'une matrice de Bayer) ; et des caméras comprenant trois capteurs CMOS (un capteur CMOS pour chaque couleur primaire). Rien n'empêche cependant d'utiliser une caméra à capteur(s) CCD ou autre.

De préférence, chaque caméra multispectrale est une caméra couleur comprenant un capteur CMOS et une matrice de filtres de couleurs, et exempte de filtre coupant les infrarouges. Il s'avère en effet qu'une telle caméra est effectivement sensible aux infrarouges et peut être adaptée pour réaliser des images dans tout domaine visible et dans tout domaine infrarouge.

Une caméra couleur de type à capteur(s) CMOS comprend trois groupes d'éléments photosensibles, chaque groupe détectant l'une des couleurs primaires. Dans certains modes de réalisation préférentiels, dans un procédé selon l'invention, chaque image dans un domaine infrarouge est réalisée avec un ajustement de la balance des blancs en fonction de la sensibilité pour chaque couleur (c'est-à-dire de chaque groupe d'éléments photosensibles à l'une des couleurs primaires) de la caméra multispectrale dans ledit domaine infrarouge. En outre, dans ces modes de réalisation, un dispositif selon l'invention pour la réalisation de chaque image dans un domaine infrarouge, ledit dispositif de commande est adapté pour ajuster la balance des blancs en fonction de la sensibilité de chaque couleur de la caméra multispectrale dans ledit domaine infrarouge. On obtient ainsi une image infrarouge de meilleure qualité.

En effet, il a été constaté que la sensibilité de chaque groupe d'éléments photosensibles d'une caméra couleur pour une longueur d'onde infrarouge varie selon la couleur de détection de ce groupe d'éléments photosensibles. Si ledit domaine infrarouge comprend une unique longueur d'onde infrarouge, la balance des blancs est ajustée en fonction de la sensibilité de chaque couleur de la caméra multispectrale pour cette longueur d'onde infrarouge. Si ledit domaine infrarouge comprend une bande de longueurs d'onde infrarouge, la balance des blancs peut être ajustée en fonction de la sensibilité de chaque couleur de la caméra multispectrale pour une longueur d'onde caractéristique, notamment la longueur d'onde centrale, de ladite bande de longueurs d'onde infrarouge.

Il est à noter cependant que rien n'empêche de prévoir en variante la réalisation d'images en infrarouge sans ajustement de la balance des blancs.

Diverses techniques d'imagerie peuvent être utilisées dans un procédé et un dispositif d'analyse optique selon l'invention, et différentes techniques d'imagerie peuvent être combinées avec une même caméra multispectrale et/ou avec différentes caméras multispectrales. En particulier, dans un procédé et un dispositif d'analyse optique selon l'invention lesdites images réalisées par une même caméra multispectrale peuvent être aussi bien des images réalisées en réflexion et/ou des images réalisées en transmission et/ou des images réalisées en diffusion.

De même, chaque domaine de longueur d'onde d'éclairage peut correspondre, ou non, à chaque domaine de longueur d'onde de prise de vues. Ainsi, par exemple, il est possible d'éclairer l'objet dans un domaine visible et de réaliser des images avec une caméra multispectrale dans ce même domaine visible ; d'éclairer l'objet dans un domaine infrarouge et de réaliser des images avec une caméra multispectrale dans ce même domaine infrarouge ; d'éclairer l'objet dans un domaine ultraviolet et de réaliser des images avec une caméra multispectrale dans un domaine visible (par fluorescence) ; etc.

Pour la réalisation d'images en réflexion la zone éclairée de surface extérieure de l'objet par au moins une source lumineuse est l'intégralité d'une face visible de la surface extérieure de l'objet par le rayonnement lumineux de cette source lumineuse, et au moins une caméra multispectrale est agencée par rapport cette source lumineuse pour réaliser des images en réflexion de l'intégralité de cette face visible. Ainsi, pour réaliser une image en réflexion, au moins une source lumineuse applique un rayonnement lumineux sur ladite portion de surface extérieure de l'objet éclairé vers laquelle la caméra multispectrale est orientée.

Pour réaliser une image en transmission, au moins une source lumineuse applique un rayonnement lumineux sur une zone de surface extérieure de l'objet diamétralement opposée à ladite portion de surface extérieure de l'objet éclairé vers laquelle la caméra multispectrale est orientée. Pour réaliser une image en diffusion, au moins une source lumineuse applique un rayonnement lumineux sur une zone, dite zone éclairée, de surface extérieure de l'objet disjointe de ladite portion de surface extérieure de l'objet éclairé vers laquelle la caméra multispectrale est orientée, la caméra multispectrale étant orientée par rapport à cette zone éclairée selon un angle de prise de vues supérieur à 90° et inférieur à 180°.

Cela étant, dans certains modes de réalisation avantageux conformes à l'invention, au moins une caméra multispectrale est agencée par rapport aux dites sources lumineuses pour réaliser des images en réflexion uniquement. En effet, dans de nombreuses applications, l'analyse optique de fruits ou légumes peut être réalisée exclusivement avec de telles images en réflexion. Tel est le cas en particulier pour l'analyse optique de fruits appartenant au groupe des pommes, des poires, des fruits à noyau (pêches, nectarines, abricots...), des tomates.

Dans d'autres modes de réalisation conformes à l'invention au moins une caméra multispectrale peut être agencée par rapport à au moins une partie des sources lumineuses pour réaliser des images en diffusion/transmission, notamment dans un domaine visible. Tel est le cas en particulier pour l'analyse optique d'agrumes. Avantageusement, au moins une autre partie des sources lumineuses peut être agencée par rapport à la caméra multispectrale pour permettre la réalisation d'images en réflexion, notamment des images réflexion dans un domaine infrarouge sous éclairage dans un domaine infrarouge et/ou des images en réflexion dans un domaine visible sous éclairage dans un domaine visible et/ou des images en réflexion dans le domaine visible sous éclairage dans un domaine ultraviolet.

Selon la technique d'imagerie concernée, la zone éclairée de surface extérieure de l'objet par une source lumineuse peut être aussi bien :
- l'intégralité d'une face visible de la surface extérieure de l'objet selon la direction de propagation dudit rayonnement lumineux émis par cette source lumineuse et appliqué sur la surface extérieure de l'objet -notamment une calotte diamétrale de l'objet lorsque ce dernier est globalement sphérique- ; tel est en particulier avantageusement le cas pour la réalisation d'images en réflexion ;
- une zone de focalisation dudit rayonnement lumineux sur la surface extérieure de l'objet, cette zone de focalisation étant plus petite que la face visible de la surface extérieure de l'objet selon la direction de propagation dudit rayonnement lumineux -notamment plus petite qu'une calotte diamétrale de l'objet lorsque ce dernier est globalement sphérique-; tel est en particulier avantageusement le cas pour la réalisation d'images en diffusion/transmission.

Cela étant, avantageusement et selon l'invention au moins une source lumineuse est agencée pour pouvoir appliquer un rayonnement lumineux sur l'intégralité d'une face visible de la surface extérieure de chaque objet éclairé par cette source lumineuse.

Par ailleurs, rien n'empêche que certaines images réalisées par une caméra multispectrale selon l'invention ne représente pas l'intégralité de la face visible de la surface extérieure de l'objet selon l'axe optique de la caméra. Ainsi, ladite portion de surface extérieure de l'objet éclairé peut, dans certains modes de réalisation et pour au moins une caméra multispectrale, correspondre à une portion de surface extérieure de l'objet plus petite qu'une face visible de cette surface extérieure selon l'axe optique de la caméra.

Cela étant, au moins une caméra multispectrale est agencée par rapport à l'objet éclairé de telle sorte que ladite portion de surface extérieure de l'objet éclairé dont des images sont réalisées par la caméra multispectrale correspond à toute la face visible de la surface extérieure de l'objet selon l'axe optique de la caméra multispectrale. Ainsi, lorsque l'objet est de forme globalement sphérique, les images réalisées par la caméra multispectrale sont des images d'une calotte diamétrale de l'objet.

Par ailleurs, ladite séquence d'éclairage et la synchronisation de l'exposition de chaque caméra multispectrale par rapport à cette séquence d'éclairage peuvent faire l'objet de nombreuses variantes de réalisation.

En particulier, dans certains modes de réalisation avantageux selon l'invention, ladite séquence d'éclairage est formée d'une succession de durées d'éclairage, une partie desdites sources lumineuses étant activée durant chaque durée d'éclairage, cette partie desdites sources lumineuses étant choisie pour éclairer chaque objet dans l'un desdits domaines de longueur d'onde d'éclairage. En outre, de préférence, les domaines de longueur d'onde d'éclairage de deux durées d'éclairage successives sont distincts l'un de l'autre.

Chaque durée d'éclairage peut être particulièrement brève, la durée d'exposition de la caméra multispectrale pendant une durée d'éclairage pouvant également être particulièrement brève, l'image réalisée pendant cette durée d'exposition pouvant être enregistrée dans une mémoire tampon de la caméra multispectrale. Ainsi, dans certains modes de réalisation avantageux conformes à l'invention, chaque durée d'éclairage de ladite séquence d'éclairage est comprise entre 0,1 ms et 5 ms -notamment entre 0,1 ms et 1,5 ms-.

De même, rien n'empêche que les différentes durées d'éclairage successives d'une séquence d'éclairage soient séparées les unes des autres par des durées d'extinction de l'intégralité desdites sources lumineuses. Ces durées d'extinction sont par exemple avantageusement comprises entre 0,05 ms et 0,5 ms -notamment de l'ordre de 0,1 ms-. Néanmoins, de préférence, les différentes durées d'éclairage d'une même séquence d'éclairage se suivent sans interruption, c'est-à-dire sans durée d'extinction entre deux durées d'éclairage.

De préférence, l'exposition d'une caméra multispectrale est déclenchée avec un retard par rapport à l'activation d'une durée d'éclairage, ce retard étant choisi pour que chaque source lumineuse de ladite partie desdites sources lumineuses de cette durée d'éclairage soit effectivement complètement allumée et active avant le déclenchement de la caméra multispectrale. En effet, les sources lumineuses, y compris les LEDs à commande ultrarapide, nécessitent un certain délai entre la réception de leur signal d'activation et leur allumage complet. Par exemple ce retard est compris entre 0,01 ms et 0,5 ms -notamment de l'ordre de 0,05 ms-.

De même, la fermeture d'exposition de la caméra multispectrale est de préférence déclenchée avec une avance par rapport à l'extinction de l'une au moins des sources lumineuses. Ainsi on assure que pour chaque durée d'éclairage, chaque source lumineuse correspondante est et reste complètement active et allumée pendant toute la durée d'exposition de la caméra multispectrale. Par exemple cette avance est comprise entre 0,01 ms et 0,5 ms -notamment de l'ordre de 0,05 ms-.

Ainsi, il est par exemple possible de réaliser avec une même caméra multispectrale des séries d'images comprenant entre deux et dix -notamment de trois à cinq- images successives d'un même objet éclairé, dans différents domaines de longueur d'onde, dont au moins une image dans un domaine visible et au moins une image dans un domaine infrarouge, lors d'une séquence d'éclairage en une durée totale de la séquence d'éclairage inférieure à 10 ms, notamment comprise entre 1 ms et 5 ms. On peut par exemple réaliser une série de quatre images successives en une durée totale de l'ordre de 3,5 ms. Le nombre et la nature de chaque image d'une même série d'images dans différents domaines de longueur d'onde sont choisis de façon à permettre l'analyse optique des fruits ou légumes à trier à partir de ces différentes images. Ils peuvent faire l'objet de très nombreuses variantes selon les critères de tri importants pour ces fruits ou légumes, qui peuvent varier d'une variété à l'autre de fruits ou légumes et/ou en fonction des besoins du client final en ce qui concerne le tri des fruits ou légumes.

En outre, les différentes sources lumineuses sont choisies de façon à être compatibles avec ladite séquence d'éclairage, et avec les différents domaines de longueur d'onde d'éclairage et avec les caractéristiques de chaque caméra multispectrale utilisée. En particulier, dans certains modes de réalisation avantageux, lesdites sources lumineuses comportent au moins une LED à commande ultrarapide. En particulier, lesdites sources lumineuses comportent au moins une LED d'éclairage en lumière blanche visible, au moins une LED d'éclairage par un rayonnement lumineux ultraviolet -notamment une LED d'éclairage ultraviolet à une longueur d'onde comprise entre 250 nm et 380 nm, par exemple 365 nm-, et au moins une LED d'éclairage en infrarouge -notamment une LED d'éclairage infrarouge à une longueur d'onde comprise entre 720 et 780 nm, par exemple 740 nm ; une LED d'éclairage infrarouge à une longueur d'onde comprise entre 800 nm et 850 nm, par exemple 810 nm ; et une LED d'éclairage infrarouge à une longueur d'onde comprise entre 900 nm et 1000 nm, par exemple 940 nm-. D'autres exemples sont possibles.

Dans certains modes de réalisation avantageux conformes à l'invention, lesdites sources lumineuses sont montées dans une chambre optique ayant une surface interne réfléchissante dont la forme est choisie en fonction de la position des sources lumineuses de façon à permettre un éclairage uniforme des objets se trouvant dans le champ optique desdites au moins une caméra multispectrale. Un tel éclairage uniforme est une illumination homogène de toute la face de chaque objet exposée au rayonnement lumineux issu de la chambre optique. En outre, au moins une caméra multispectrale est orientée de façon à réaliser des images en réflexion de toute la face de chaque objet ainsi éclairé.

Un procédé et un dispositif d'analyse optique selon l'invention permettent en particulier l'analyse optique des fruits ou légumes alors même qu'ils sont entraînés en déplacement par un convoyeur -notamment un convoyeur à haute vitesse, par exemple un convoyeur permettant de transporter plus de dix objets par seconde, en particulier jusqu'à cinquante objets par seconde, voire plus, devant chaque caméra multispectrale-. Ainsi, dans certains modes de réalisation d'un procédé selon l'invention, chaque objet éclairé est entraîné en déplacement par un convoyeur au cours de ladite séquence d'éclairage.

De même, un procédé et un dispositif d'analyse optique selon l'invention permettent l'analyse optique des fruits ou légumes alors qu'ils sont entraînés en rotation sur eux-mêmes. Ainsi, dans certains modes de réalisation d'un procédé selon l'invention, chaque objet éclairé est entraîné en rotation sur lui-même au cours de la dite séquence d'éclairage.

L'invention permet de réduire considérablement la complexité, le nombre et l'encombrement que chaque poste d'analyse optique d'un dispositif de tri automatique de fruits ou légumes. En particulier, dans certains modes de réalisation d'un dispositif de tri automatique de fruits ou légumes selon l'invention chaque poste d'analyse optique comporte moins de quatre caméras multispectrales -notamment une caméra multispectrale ou deux caméras multispectrales- par ligne de convoyage. Il s'avère en effet qu'il est possible de réaliser avec une seule et même caméra multispectrale l'intégralité des images d'une même portion de surface extérieure des objets nécessaires à l'analyse optique des fruits ou légumes en vue de leur tri automatique : calibre, couleur, défauts internes, défauts externes, et fermeté. Par exemple, lorsque les objets sont globalement symétriques de révolution -notamment globalement sphériques-, ce qui est le cas de la plupart des fruits ou légumes, chaque poste d'analyse optique peut comporter, pour chaque ligne de convoyage, uniquement une caméra multispectrale (ou deux caméras multispectrales décalées latéralement et inclinées par rapport à la verticale, une de chaque côté de la ligne) pour réaliser des prises de vues de l'intégralité de la surface extérieure des objets au cours de leur passage dans le poste d'analyse optique, chaque objet étant entraîné en rotation d'au moins 180° sur lui-même entre son entrée dans le champ de la caméra multispectrale et sa sortie du champ de la caméra multispectrale.

Dans certains modes de réalisation de l'invention il est même possible de prévoir que le dispositif de tri automatique comporte un unique poste d'analyse optique. Tel est en particulier le cas pour le tri automatique de fruits ou légumes choisis dans le groupe constitué des pommes, poires, kiwis, agrumes, tomates, pêches, abricots, nectarines, prunes, kakis, avocats, mangues, grenades, melons cantaloup, myrtilles, cerises). D'autres exemples sont possibles.

L'invention concerne également un procédé et un dispositif d'analyse optique de fruits ou légumes, ainsi qu'un dispositif de tri automatique de fruits ou légumes caractérisés en combinaison par tout ou partie des caractéristiques mentionnées ci-dessus ou ci-après.

D'autres buts, caractéristiques et avantages de l'invention apparaîtront à la lecture de la description suivante de certains de ses modes de réalisation donnée à titre non limitatif et qui se réfère aux figures annexées dans lesquelles :
- la figure 1 est un exemple de spectre de sensibilité d'une caméra monochrome utilisée dans un dispositif d'analyse optique de l'état de la technique,
- la figure 2 est un exemple de spectre de sensibilité d'une caméra couleur utilisée dans un dispositif d'analyse optique de l'état de la technique,
- la figure 3 est un exemple de spectre de sensibilité d'une caméra couleur sensible aux infrarouges pouvant être utilisée à titre de caméra multispectrale dans un procédé et un dispositif d'analyse optique selon l'invention,
- la figure 4 est une vue schématique en élévation d'un poste d'analyse optique d'un dispositif de tri automatique selon un mode de réalisation de l'invention,
- la figure 5 est une vue schématique en coupe transversale selon la ligne V-V de la figure 4,
- la figure 6 est un chronogramme d'un exemple de séquence d'éclairage et de prise de vues d'un procédé d'analyse optique selon l'invention,
- la figure 7 est un schéma synoptique d'étapes au cours d'une séquence d'éclairage d'un mode de réalisation d'un procédé d'analyse optique selon l'invention,
- la figure 8 est un schéma synoptique des caméras, des sources lumineuses et d'un dispositif de commande des caméras et des sources lumineuses d'un dispositif d'analyse optique selon l'invention pour une ligne de convoyage d'objets.

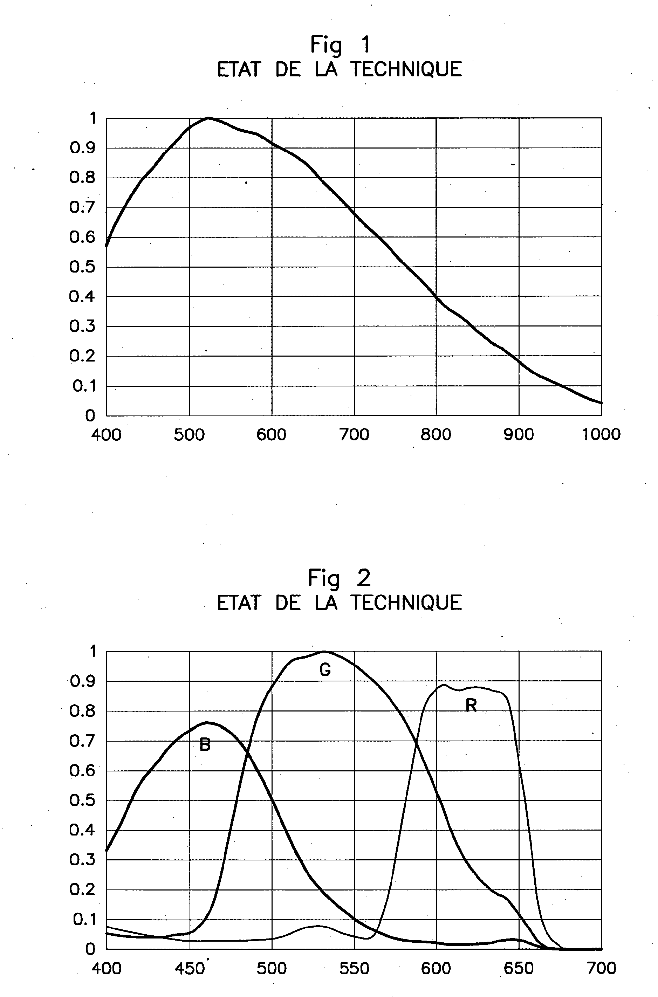

La figure 1 est un spectre de sensibilité d'une caméra monochrome comprenant un capteur CMOS classiquement utilisée dans l'état de la technique pour l'analyse optique en vue du tri automatique de fruits ou légumes par imagerie en infrarouge et/ou en ultraviolet. Comme on le voit, la sensibilité de la caméra dans le domaine infrarouge n'est pas nulle, mais est relativement faible. Il en va de même dans le domaine ultraviolet. La figure 2 est un spectre de sensibilité d'une caméra couleur comprenant un capteur CMOS et une matrice de filtres de Bayer, ainsi qu'un filtre coupant les infrarouges, classiquement utilisée dans l'état de la technique pour l'analyse optique en vue du tri automatique de fruits ou légumes par imagerie dans le domaine visible. Comme on le voit, une telle caméra couleur est parfaitement insensible dans le domaine infrarouge.

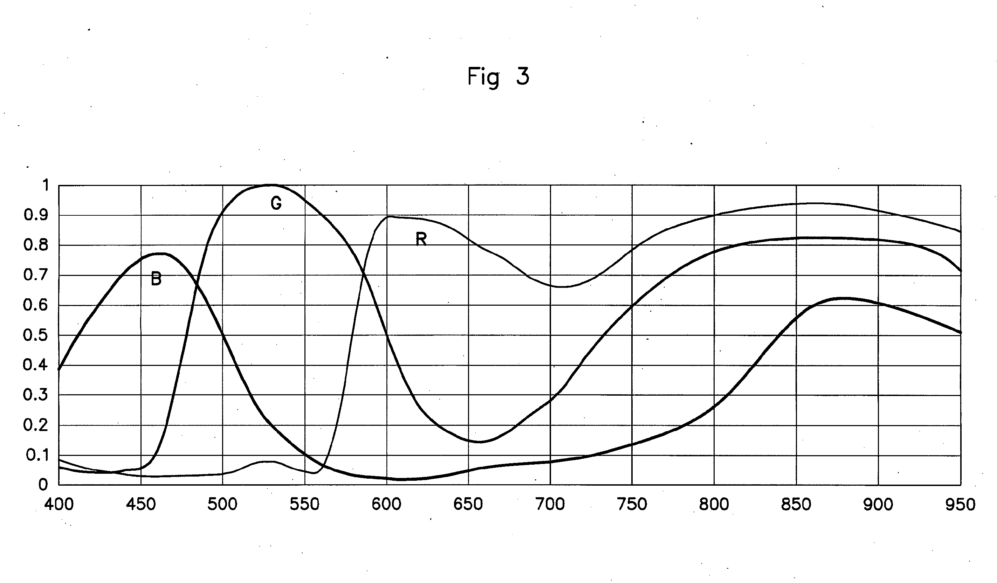

L'inventeur a par contre constaté avec surprise qu'une caméra couleur exempte de filtre coupant les infrarouges est en réalité particulièrement bien sensible dans le domaine infrarouge, comme cela est visible sur la figure 3, et peut donc être utilisée aussi bien dans le domaine visible que dans le domaine infrarouge, permettant de simplifier considérablement les postes d'analyse optique dans les dispositifs de tri automatique de fruits ou légumes. L'invention consiste donc à utiliser une même caméra couleur pour réaliser à la fois des images dans au moins un domaine visible et des images dans au moins un domaine infrarouge, et éventuellement des images dans au moins un domaine ultraviolet.

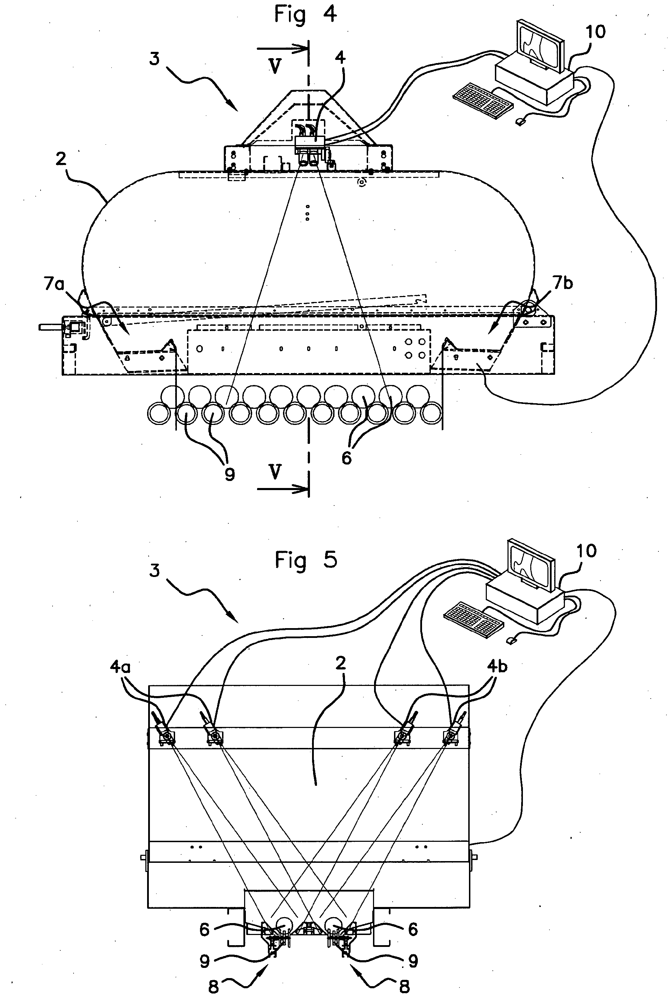

Un exemple de mode de réalisation d'un dispositif 3 d'analyse optique conforme à l'invention est représenté sur les figures 4 et 5. Ce dispositif d'analyse optique constitue un poste d'analyse optique d'un dispositif de tri automatique dont les caractéristiques générales sont bien connues en elles-mêmes (cf. par exemple US 5626238) et qui peut faire l'objet de très nombreuses variantes de réalisation, l'invention étant applicable à toutes ces variantes de réalisation, sans limitation, dès lors que le dispositif de tri automatique permet d'entraîner une pluralité d'objets 6 constitués de fruits ou des légumes en translation horizontale les uns à la suite des autres en regard du dispositif d'analyse optique, sur des supports 9 agencés selon au moins une ligne de convoyage 8, en général selon plusieurs lignes de convoyage 8 parallèles de fruits ou légumes comme dans l'exemple représenté.

De préférence, les supports 9 sont des supports tournants tels que des rouleaux entraînés non seulement en translation longitudinale, mais également en rotation sur eux-mêmes autour d'axes de rotation transversaux permettant d'entraîner les objets 6 en rotation sur eux-mêmes lorsqu'ils passent à travers le dispositif d'analyse optique. En effet, en général, les fruits ou légumes présentent au moins une symétrie de révolution et peuvent donc être entraînés en rotation sur eux-mêmes pour permettre de réaliser une imagerie de l'intégralité de leur surface extérieure par les différentes images acquises successivement d'un même objet 6 en déplacement le long de la ligne de convoyage. Les rouleaux sont par exemple formés d'une pluralité de disques parallèles, deux rouleaux successifs selon la direction longitudinale d'entraînement en translation définissant un logement de réception pour un objet 6.

Le dispositif 3 d'analyse optique selon l'invention comprend, dans l'exemple représenté, deux dispositifs 7a, 7b d'éclairage dont un 7a situé en amont et un situé 7b en aval. Les deux dispositifs 7a, 7b d'éclairage sont identiques et comprennent chacun une pluralité de sources lumineuses formées de LEDs (diodes électroluminescentes) émettant des rayonnements lumineux dans différents domaines de longueur d'onde d'éclairage.

Par exemple, chaque dispositif 7a, 7b d'éclairage comprend au moins une LED, dite LED1, émettant de la lumière blanche dans le domaine visible ; au moins une LED, dite LED2, émettant un rayonnement lumineux dans un domaine infrarouge à une longueur d'onde comprise entre 720 et 780 nm, par exemple centré sur 740 nm ; au moins une LED, dite LED3, émettant un rayonnement lumineux dans un domaine infrarouge à une longueur d'onde comprise entre 800 nm et 850 nm, par exemple centré sur 810 nm ; au moins une LED, dite LED4, émettant un rayonnement lumineux dans un domaine infrarouge à une longueur d'onde comprise entre 900 nm et 1000 nm, par exemple centré sur 940 nm ; au moins une LED, dite LED5, émettant un rayonnement lumineux dans un domaine ultraviolet à une longueur d'onde comprise entre 250 nm et 380 nm, par exemple centré sur 365 nm. De préférence, chaque dispositif 7a, 7b d'éclairage comprend plusieurs LEDs allumées simultanément pour chaque domaine de longueur d'onde d'éclairage.

Les sources lumineuses LED1, LED2, LED3, LED4, LED5 sont disposées au-dessus de la ligne de convoyage 8 et orientées vers le haut, de sorte qu'elles n'apportent aucun éclairage direct sur les objets 6. Les sources lumineuses LED1, LED2, LED3, LED4, LED5 sont en revanche montées dans une chambre 2 optique dont la surface interne est réfléchissante. La chambre 2 optique présente en outre une base ouverte de sorte que le rayonnement émis par les sources lumineuses est réfléchi par la surface interne de la chambre optique et est dirigé vers les fruits ou légumes défilant au-dessous de la chambre 2 optique.

Le dispositif 3 d'analyse optique selon l'invention comprend au moins une caméra 4 couleur sensible aux infrarouges. Il est à noter que dans un dispositif selon l'invention, il est possible de prévoir une et une seule caméra 4.

Dans l'exemple représenté d'un poste optique pour deux lignes 8 de convoyage parallèles, le dispositif 3 d'analyse optique comprend de préférence quatre caméras 4a, 4b disposées au-dessus des deux lignes 8 de convoyage de fruits ou légumes, en partie supérieure de la chambre 2 optique, c'est-à-dire deux caméras 4a, 4b pour chaque ligne 8 de convoyage.

Les caméras 4a, 4b sont agencées avec leur axe optique légèrement incliné par rapport à la verticale au-dessus des lignes 8 de convoyage. Elles sont disposées respectivement d'un côté et de l'autre de la ligne 8 de convoyage dont elles réalisent des images, de sorte qu'elles acquièrent une image sensiblement différente de chaque objet 6, une première caméra 4a permettant d'obtenir une image d'une portion supérieure et d'une première face latérale de l'objet entraîné par la ligne 8 de convoyage, et une seconde caméra 4b permettant d'obtenir une image d'une portion supérieure et d'une seconde face latérale (opposée à la première face latérale) de l'objet entraîné par la ligne 8 de convoyage.

Chaque caméra 4a, 4b présente un champ optique qui couvre une longueur suffisamment importante de la ligne 8 de convoyage correspondante de telle sorte qu'elle puisse réaliser une pluralité d'images de chaque objet 6 entraîné par cette ligne 8 de convoyage, dont au moins deux images de deux portions diamétralement opposées de chaque objet 6. Chaque image représente plusieurs objets 6 successifs longitudinalement le long de la ligne 8 de convoyage, le traitement des images permettant d'identifier chaque objet dans chaque image de façon bien connue en soi. En outre, les caméras réalisent chacune une pluralité de séries d'images successives d'un même objet au cours de son passage en regard du dispositif 3 d'analyse optique. En pratique, il est possible de réaliser par exemple entre 5 et 50 séries d'images de chaque objet, typiquement de l'ordre de 10 séries d'images de chaque objet au cours de son transport en regard de chaque caméra, chaque image correspondant à une portion différente de la surface extérieure de l'objet, ce dernier étant entraîné en rotation.

Rien n'empêche de prévoir en variante d'autres dispositions de caméras, par exemple avec une même caméra focalisée longitudinalement sur un seul objet 6, le dispositif 3 d'analyse optique comprenant alors un nombre de caméras successives longitudinalement suffisant pour permettre de réaliser des séries d'images de toute la surface extérieure de chaque objet et/ou une même caméra multispectrale permettant de réaliser des images d'objets entraînés par plusieurs lignes 8 de convoyage parallèles, c'est-à-dire des images dans lesquelles des objets sont juxtaposés latéralement.

Comme représenté à la figure 4, la forme de la surface interne de la chambre 2 optique est avantageusement choisie en fonction de la position des sources lumineuses des dispositifs 7a, 7b d'éclairage de façon à permettre un éclairage uniforme des objets 6 se trouvant dans le champ optique des caméras 4a, 4b du dispositif 3 d'analyse optique.

Chaque caméra 4a, 4b est une caméra couleur sensible aux infrarouges, par exemple choisie dans le groupe des caméras comprenant un capteur CMOS (avec une matrice de filtres de couleurs telle qu'une matrice de Bayer mais exempte de filtre coupant les infrarouges) ; des caméras comprenant trois capteurs CMOS (un capteur CMOS pour chaque couleur primaire) et exempte de filtre coupant les infrarouges.

Comme représenté, les caméras 4a, 4b sont disposées au-dessus des lignes de convoyage 8, avec leur axe optique orienté vers le bas incliné vers l'une des lignes 8 de convoyage.

Chaqué caméra 4a, 4b est dotée d'une mémoire 42a, respectivement 42b, interne permettant de mémoriser une pluralité d'images réalisées successivement par la caméra. Ainsi, chaque caméra peut être commandée en mode rafale selon une séquence à haute vitesse pour réaliser successivement plusieurs images de chaque objet présent dans son champ optique, les différentes images d'une même série d'images pouvant être réalisées dans différents domaines de longueur d'onde de prise de vues et mémorisées en temps réel dans la mémoire de la caméra. De préférence, on choisit chaque caméra 4a, 4b de telle sorte que sa mémoire interne soit suffisante pour permettre de mémoriser plusieurs images réalisées successivement par la caméra au cours d'une séquence d'éclairage, ces différentes images correspondant à une série d'images dans différents domaines de longueur d'onde de prise de vues, cette série d'images permettant d'analyser optiquement les différents critères de tri nécessaire au tri automatique des objets. En outre, de préférence, chaque caméra 4a, 4b est une caméra à haute définition, c'est-à-dire à plus de 1 millions de pixels. Par exemple, de très bons résultats ont été obtenus avec des caméras comprenant un capteur CMOS de 1920 x 1200 pixels.

Par exemple, chaque série d'images peut comporter:
- une image réalisée en réflexion alors que l'objet est éclairé par de la lumière blanche (par chaque LED1),
- une image réalisée en réflexion alors que l'objet est éclairé dans un domaine infrarouge d'éclairage (par chaque LED2) à une longueur d'onde comprise entre 720 et 780 nm, par exemple centré sur 740 nm, ,
- une image réalisée en réflexion alors que l'objet est éclairé dans un domaine infrarouge d'éclairage (par chaque LED3) à une longueur d'onde comprise entre 800 nm et 850 nm, par exemple centré sur 810 nm,
- une image réalisée en réflexion alors que l'objet est éclairé dans un domaine infrarouge d'éclairage (par chaque LED4) à une longueur d'onde comprise entre 900 nm et 1000 nm, par exemple centré sur 940 nm,
- une image réalisée en réflexion alors que l'objet est éclairé dans un domaine ultraviolet d'éclairage (par chaque LED5) à une longueur d'onde comprise entre 250 nm et 380 nm, par exemple centré sur 365 nm.

Ces différentes images permettent notamment de détecter et de discriminer des caractéristiques, externes ou internes, maladies ou défauts, choisis dans le groupe formé des tavelures, de la maladie des tâches amères, des diverses formes de pourriture, de l'échaudage superficiel, des parasites tels que le *Gloesporium*, des coups de soleil, des impacts de grêle, des mâchures, des piqûres, des perforations et des rugosités superficielles de la peau telles que le russeting.

D'autres exemples sont possibles, et en particulier rien n'empêche de prévoir de réaliser également des images en diffusion/transmission, par exemple en focalisant un rayonnement lumineux dans le domaine visible sur une zone de la surface extérieure d'un objet et en réalisant une image avec une caméra d'une portion de la surface extérieure de l'objet disjointe de la zone de focalisation, notamment avec un axe optique de la caméra formant un angle compris entre 90° et 180° par rapport à l'axe du rayonnement lumineux. Pour ce faire, il n'est pas nécessaire d'augmenter le nombre de caméras du dispositif d'analyse optique, et il suffit par exemple de prévoir une source lumineuse sur le côté et/ou en dessous de chaque ligne 8 de convoyage.

Il est à noter que chaque caméra 4a, 4b étant une caméra couleur sensible aux infrarouges exempte de filtres autres que ceux nécessaires à la détection des couleurs (notamment matrice de filtres de Bayer), chaque image réalisée couvre l'ensemble du spectre de sensibilité de la caméra. Ainsi, lorsque les objets sont éclairés en lumière blanche, l'image formée par la caméra est une image en couleur dans le domaine visible de prise de vues. De même, lorsque les objets sont éclairés dans un domaine de longueur d'onde d'éclairage compris dans les infrarouges, l'image formée par la caméra est une image réalisée dans le même domaine de longueur d'onde infrarouge. Et lorsque les objets sont éclairés par un rayonnement lumineux ultraviolet, l'image formée par la caméra est une représentation en couleur de la fluorescence des objets dans le domaine visible.

Chaque source lumineuse LED1, LED2, LED3, LED4, LED5 et chaque caméra 4a, 4b est commandée à partir d'un système 10 informatique d'analyse des images acquises par les caméras 4a, 4b. Ce système 10 informatique peut faire l'objet de toutes variantes dès lors qu'il est adapté et programmé pour :
- commander l'allumage et l'extinction de chaque source lumineuse selon une séquence d'éclairage prédéterminée pour permettre la réalisation de chaque série d'images,
- recevoir les différentes images acquises par les différentes caméras,
- analyser ces images et en déduire des critères de tri automatique des objets,
- commander le dispositif de tri automatique en fonction des critères de tri résultant de l'analyse optique des objets ainsi réalisée.

Un tel système 10 informatique peut comporter un dispositif informatique unique tel qu'un ordinateur comme représenté sur les figures, ou une pluralité de dispositifs informatiques et/ou ressources informatiques et/ou terminaux et/ou périphériques distants les uns des autres et reliés en réseau. Le système 10 informatique peut également être formé de plusieurs dispositifs informatiques distincts les uns des autres et non reliées, chacun étant dédié à une fonction spécifique : par exemple un dispositif informatique pour commander le dispositif d'analyse optique, et donc réaliser l'imagerie et l'analyse optique des objets ; et un autre dispositif informatique pour le tri automatique des objets. Le système 10 informatique est par ailleurs adapté pour pouvoir exécuter un programme d'ordinateur ou une pluralité de programmes d'ordinateur, notamment pour la mise en œuvre d'un procédé selon l'invention.

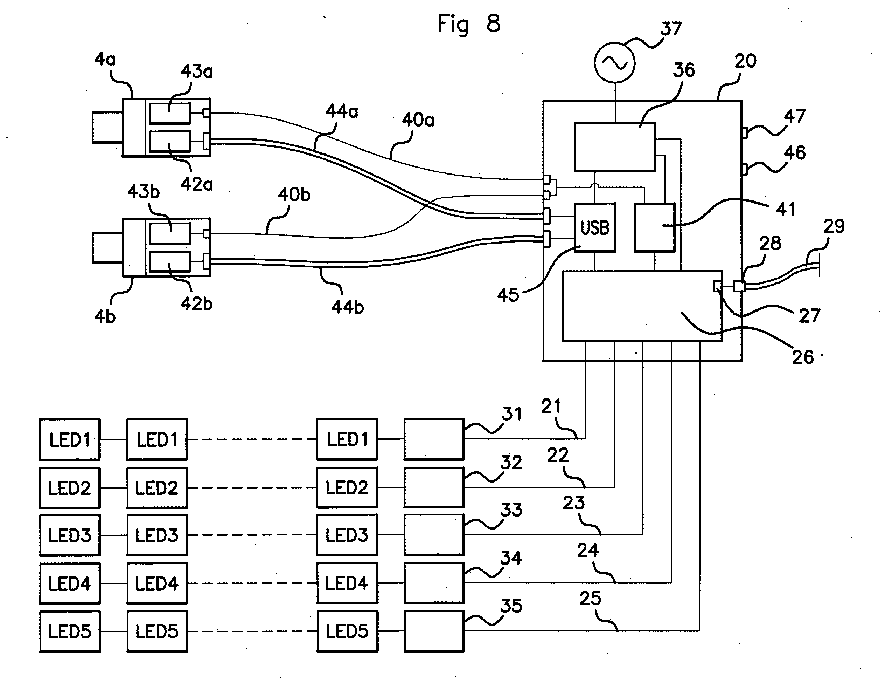

Le système 10 informatique comprend une carte 20 électronique de commande (figure 8) des sources lumineuses LED1, LED2, LED3, LED4, LED5 et des caméras 4a, 4b. Sur la figure 8, on a représenté uniquement deux caméras 4a, 4b orientées vers la même ligne 8 de convoyage. Chaque source lumineuse LED1, LED2, LED3, LED4, LED5 est reliée à la carte 20 électronique de commande par un câble 21, 22, 23, 24, 25 d'alimentation propre respectivement à chaque groupe de LEDs devant être activées simultanément, c'est-à-dire correspondant à un même domaine de longueur d'onde d'éclairage, l'allumage et l'extinction des différentes sources lumineuses étant commandés par cette carte 20 électronique de commande. Les différentes LEDs de chaque groupe de LEDs sont de préférence reliées à une même carte 31, 32, 33, 34, 35 de commande (à haute vitesse) et d'alimentation propre respectivement à ce groupe de LEDs et recevant le câble 21, 22, 23, 24, 25 d'alimentation.

La carte 20 électronique de commande comprend un circuit intégré 26, qui est par exemple un circuit intégré programmable (FPGA) auquel les différents câbles 21, 22, 23, 24, 25 d'alimentation des LEDS sont directement connectés. Ce circuit intégré 26 présente également un port d'entrée 27 relié à un connecteur 28 réseau de la carte 20 de commande auquel peut être branché un câble 29 tel qu'un câble Ethernet en provenance d'une carte mère du système 10 informatique.

La carte 20 électronique de commande comprend également un circuit 36 d'alimentation électrique pouvant être relié à une source 37 d'énergie électrique, ce circuit 36 d'alimentation électrique alimentant les différents composants de la carte 20 électronique de commande.

Chaque caméra 4a, 4b est aussi reliée à la carte 20 électronique de commande par un câble 40a, respectivement 40b, électrique propre à chaque caméra 4a, 4b, de façon à pouvoir être commandée par cette dernière pour son déclenchement en vue de la prise d'une image ou d'une série d'images. Chaque câble 40a, 40b électrique permet de transmettre à la caméra un signal de déclenchement TRIG CAM élaboré par un circuit 41 de formatage sur commande du circuit intégré 26. Ainsi, le circuit intégré 26 peut déclencher chaque caméra 4a, 4b à un instant défini précisément en délivrant un signal au circuit 41 de formatage, ce dernier mettant en forme le signal de déclenchement TRIG CAM et le délivrant sur les câbles 40a, 40b électriques.

De préférence, chaque caméra 4a, 4b comprend un circuit électronique séquenceur 43a, respectivement 43b programmable permettant de programmer une séquence prédéterminée de prises de vues correspondant à une série d'images telle que définie ci-dessus, c'est-à-dire correspondant elle-même à une séquence d'éclairage prédéterminée. Le signal de déclenchement TRIG CAM permet donc de déclencher le séquenceur de la caméra et d'initier une séquence de prise de vues préalablement programmée par chaque caméra 4a, 4b. Le séquenceur 43a, 43b déclenche lui-même successivement chaque prise de vue, en maintenant l'exposition de la caméra pendant une durée correspondant à la capture d'une image. Également, le séquenceur 43a, 43b est programmé de façon à commander la caméra selon différents paramètres photographiques adaptés à la réalisation de chaque image, notamment en fonction du domaine de longueur d'onde considéré.

En particulier, pour la réalisation d'images dans un domaine infrarouge le séquenceur 43a, 43b ajuste la balance des blancs de la caméra de façon à optimiser la qualité de l'image, en fonction de la sensibilité dans le domaine infrarouge de chaque groupe d'éléments photosensibles à l'une des couleurs primaires de la caméra. En effet, comme on le voit figure 3, les différents groupes d'éléments photosensibles dans les différentes couleurs primaires de la caméra n'ont pas tous la même sensibilité en infrarouge. Il est donc avantageux de rééquilibrer ces différences de sensibilité, et ce simplement en ajustant la balance des blancs de la caméra avant de capturer l'image infrarouge correspondante. Cet ajustement peut être effectué en mesurant préalablement expérimentalement ces différences de sensibilité pour chaque longueur d'onde infrarouge d'éclairage, c'est-à-dire à partir de la connaissance du spectre tel que représenté figure 3.

Le système 10 informatique, sa carte 20 électronique de commande et chaque séquenceur 43a, 43b de chaque caméra multispectrale constituent ensembles un dispositif de commande de chaque séquence d'éclairage et de chaque séquence de prises de vues permettant de réaliser chaque série d'images par chaque caméra multispectrale.

Chaque caméra 4a, 4b est aussi reliée à la carte 20 électronique de commande par un câble 44a, respectivement 44b USB3 à haute vitesse, d'une part pour son alimentation électrique, d'autre part pour la transmission des différentes images d'une série d'images à destination du système 10 informatique. Ces câbles USB3 sont reliés à un circuit 45 de connexion multiports USB3 de la carte 20 électronique de commande.

La carte 20 électronique de commande comprend également avantageusement un bouton 46 permettant de l'allumer ou de l'éteindre, et un voyant 47 lumineux permettant de signaler tout éventuel dysfonctionnement.

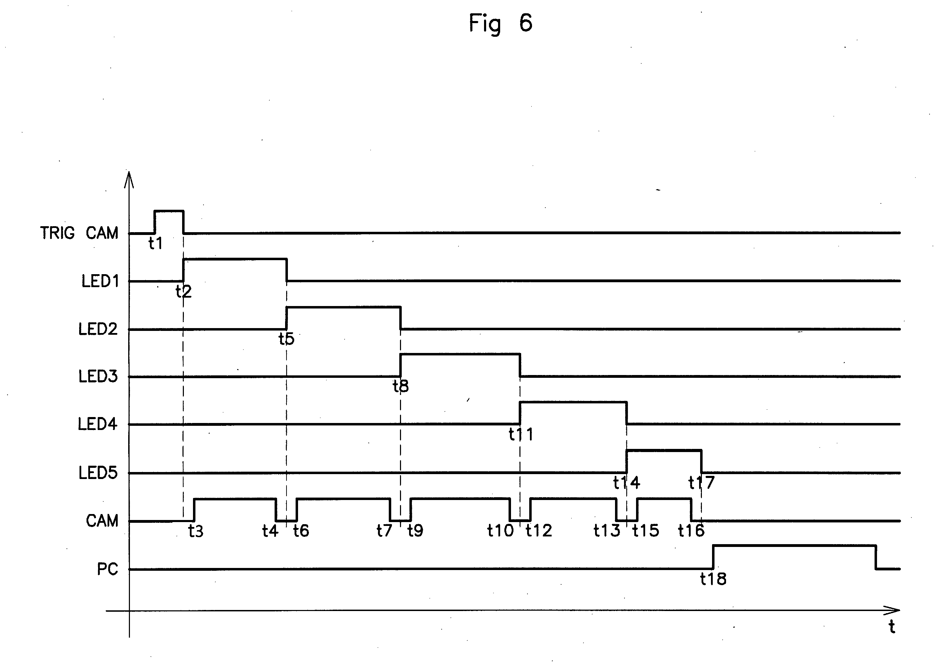

La figure 6 représente un exemple de chronogramme d'une séquence d'éclairage selon l'invention permettant de réaliser une série d'images telle que mentionnée ci-dessus.

À l'instant t1, la carte 20 électronique de commande émet le signal de déclenchement TRIG CAM des caméras 4a, 4b. Sur réception de ce signal de déclenchement, le séquenceur de chaque caméra exécute la séquence pour laquelle il a été programmé, et qui correspond au signal CAM représenté figure 6. La première prise de vue est déclenchée à un instant t3 après une faible durée d'attente après l'instant t1 de réception du signal de déclenchement.

Également, la carte 20 électronique de commande déclenche la séquence d'éclairage des différentes sources lumineuses LED1, LED2, LED3, LED4, LED5 selon les signaux correspondants représentés figure 6. Comme on le voit, les sources lumineuses LED1 sont allumées à un instant t2 précédant immédiatement l'instant t3 d'une durée suffisante pour permettre à chaque LED correspondante de s'allumer pleinement, suite à l'activation de la carte de commande et d'alimentation correspondante.

Chaque caméra 4a, 4b capture l'image pendant une durée dépendant de la taille dé l'image (nombre de pixels en ligne et nombre de pixels en colonne) et du temps d'intégration de la caméra. À la fin de cette durée à l'instant t4, une première image est capturée par la caméra et est mémorisée dans sa mémoire 42a, 42b. Il est à noter que pendant toute la durée de la capture de l'image par la caméra, les sources lumineuses LED1 restent allumées pleinement. À un instant t5 subséquent à l'instant t4, les sources lumineuses LED1 sont éteintes, et les sources lumineuses LED2, correspondant à un autre domaine de longueur d'onde d'éclairage, sont allumées. L'instant t5 est légèrement décalé de l'instant t4 de fin de capture de l'image par la caméra d'une durée aussi faible que possible, mais suffisante pour permettre d'assurer que les sources lumineuses restent allumées pendant toute cette capture.

Le cycle se répète pour la capture de quatre autres images sous éclairage successivement par les sources lumineuses LED2, puis LED3, puis LED4, puis LED5. La capture de la deuxième image commence à l'instant t6 et se termine à l'instant t7. Les sources lumineuses LED2 sont éteintes et les sources lumineuses LED3 sont allumées à l'instant subséquent t8. La capture de la troisième image commence à l'instant t9 et se termine à l'instant t10. Les sources lumineuses LED3 sont éteintes et les sources lumineuses LED4 sont allumées à l'instant subséquent t11. La capture de la quatrième image commence à l'instant t12 et se termine à l'instant t13. Les sources lumineuses LED4 sont éteintes et les sources lumineuses LED5 sont allumées à l'instant subséquent t14. La capture de la cinquième image commence à l'instant t15 et se termine à l'instant t16. Les sources lumineuses LED5 sont éteintes à l'instant subséquent t17.

Après capture de l'intégralité des images d'une même série, toutes ces images peuvent être ensuite transmises, à partir de l'instant subséquent t18 défini par le séquenceur de chaque caméra, au système 10 informatique via les câbles USB3 44a, 44b.

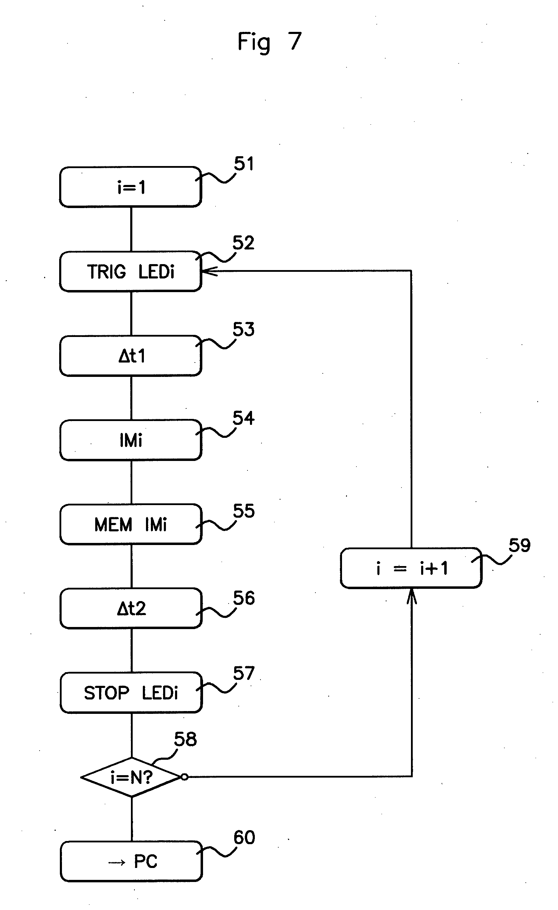

La figure 7 représente une variante dans laquelle la carte 20 électronique de commande déclenche non seulement l'allumage des différentes sources lumineuses, mais également chaque caméra si ces dernières sont dépourvues de séquenceur, pour une séquence d'éclairage et de capture d'une série d'images conforme au chronogramme de la figure 6.

Lors de la première étape 51 un index i représentant chaque groupe LEDi de LEDs est initialisé à 1. Dans l'exemple mentionné ci-dessus, i varie de 1 à N = 5. Lors de l'étape 52 subséquente les sources lumineuses LEDi sont allumées. Après une attente Δt1 (égale à t3-t2 pour LED1) pendant l'étape 53, les images IMi par les différentes caméras 4a, 4b sont capturées (entre t3 et t4 pour LED1) lors de l'étape subséquente 54 puis mises en mémoire de chaque caméra lors de l'étape 55. Après une attente Δt2 (égale à t5-t4 pour LED1), pendant l'étape 56, les sources lumineuses LEDi sont éteintes lors de l'étape subséquente 57, puis un test 58 est exécuté sur l'index i pour déterminer si la valeur maximale N de cet index est atteinte ou non. Dans la négative, l'index i est incrémenté de 1 lors de l'étape 59 et le procédé est réitéré à partir de l'étape 52 pour l'allumage des sources lumineuses du groupe suivant. Dans l'affirmative, les images sont transmises au système 10 informatique lors de l'étape 60.

L'invention permet ainsi une analyse optique des objets par utilisation de caméras couleur sensibles aux infrarouges multispectrales en mode rafale permettant de réaliser des séries d'images successives à très haute vitesse. Chaque série d'images réalisée par une caméra multispectrale selon l'invention correspond à une image réalisée par une caméra dans l'état de la technique. Mais au lieu de nécessiter une pluralité de caméras pour réaliser ces différentes images, l'invention permet de n'utiliser qu'une ou deux caméras multispectrales pour chaque ligne de convoyage.

Il va de soi que l'invention peut faire l'objet de nombreuses variantes autres que les modes de réalisation décrits ci-dessus et représentés sur les figures.

## Revendications

1. Procédé d'analyse optique d'objets appartenant au groupe des fruits et légumes dans lequel des images représentatives des objets sont réalisées dans différents domaines de longueur d'onde de prise de vue et sont traitées en vue du tri automatique des objets,
dans lequel :
- une pluralité de sources lumineuses formées de diodes électroluminescentes (LED1, LED2, LED3, LED4, LED5) sont agencées pour pouvoir appliquer chacune un rayonnement lumineux sur au moins une portion de surface extérieure d'au moins un objet, dit objet éclairé (6), les différentes sources lumineuses étant adaptées pour pouvoir appliquer sélectivement sur chaque objet éclairé des rayonnements lumineux dans différents domaines de longueur d'onde d'éclairage,
- le rayonnement lumineux issu d'au moins une source lumineuse est appliqué sur l'intégralité d'une face visible de la surface extérieure de chaque objet éclairé par cette source lumineuse,
- les sources lumineuses sont commandées selon une séquence d'éclairage prédéterminée de chaque objet éclairé, successivement selon lesdits différents domaines de longueur d'onde d'éclairage, chaque objet éclairé (6) étant entraîné en rotation sur lui-même et en déplacement par un convoyeur au cours de ladite séquence d'éclairage,
- des images sont réalisées par au moins une caméra (4, 4a, 4b) couleur sensible aux rayonnements lumineux dans le domaine visible et de longueur d'onde comprise entre 380 nm et 700 nm et aux rayonnements lumineux dans le domaine des infrarouges et de longueur d'onde comprise entre 700 nm et 1000 nm, dite caméra multispectrale (4, 4a, 4b), ayant des groupes d'éléments photosensibles aptes à détecter chacun l'une des couleurs primaires, étant orientée vers une portion de surface extérieure d'au moins un objet éclairé correspondant à toute la face visible de la surface extérieure de l'objet selon l'axe optique de la caméra multispectrale, et dont l'exposition est commandée en synchronisme avec ladite séquence d'éclairage de façon à réaliser avec cette même caméra multispectrale une pluralité d'images dans différents domaines de longueur d'onde de prise de vues de ladite portion de surface extérieure d'au moins un objet éclairé (6), dont au moins une image dans un domaine visible et au moins une image dans un domaine infrarouge,
- chaque caméra (4, 4a, 4b) multispectrale est choisie dans le groupe des caméras comprenant un capteur CMOS avec une matrice de filtres de couleurs, exemptes de filtre coupant les infrarouges ; et des caméras comprenant trois capteurs CMOS, un capteur CMOS pour chaque couleur primaire, exemptes de filtre coupant les infrarouges, et
- chaque image dans un domaine infrarouge est réalisée avec un ajustement de la balance des blancs en fonction de la sensibilité pour chaque couleur dans ledit domaine infrarouge, de chaque groupe d'éléments photosensibles de la caméra multispectrale (4, 4a, 4b) dans ledit domaine infrarouge,
- chaque caméra multispectrale (4, 4a, 4b) étant dotée d'une mémoire tampon de mémorisation des images, ladite pluralité d'images de ladite portion de surface extérieure est réalisée par une même caméra multispectrale à la vitesse maximale d'acquisition d'images par ladite caméra multispectrale, et est enregistrée dans la mémoire (42a, 42b) tampon de ladite caméra multispectrale.

2. Procédé selon la revendication 1, dans lequel la pluralité d'images de ladite portion de surface extérieure réalisée par une même caméra (4, 4a, 4b) multispectrale qui comprend entre deux et dix - notamment de trois à cinq - images successives du même objet éclairé, dans différents domaines de longueur d'onde, dont au moins une image dans un domaine visible et au moins une image dans un domaine infrarouge, ladite pluralité d'images étant réalisée lors d'une séquence d'éclairage en une durée totale de la séquence d'éclairage inférieure à 10 ms, notamment comprise entre 1 ms et 5 ms.

3. Procédé selon l'une quelconque des revendications 1 et 2, dans lequel la séquence d'éclairage est formée d'une succession de durées d'éclairage, une parties desdites sources lumineuses (LED1, LED2, LED3, LED4, LED5) étant activée durant chaque durée d'éclairage, cette partie desdites sources lumineuses étant choisie pour éclairer chaque objet dans l'un desdits domaines de longueur d'onde d'éclairage, en ce que chaque durée d'éclairage est comprise entre 0,1 ms et 5 ms, et en ce que l'exposition d'une caméra (4, 4a, 4b) multispectrale est déclenchée avec un retard par rapport à l'activation d'une durée d'éclairage, ce retard étant choisi pour que chaque source lumineuse de ladite partie desdites sources lumineuses (LED1, LED2, LED3, LED4, LED5) de cette durée d'éclairage soit effectivement complètement allumée et active avant le déclenchement de la caméra multispectrale.

4. Procédé selon la revendication 3, dans lequel les domaines de longueur d'onde d'éclairage de deux durées d'éclairage successives de la séquence d'éclairage sont distincts l'un de l'autre.

5. Procédé selon l'une quelconque des revendications 3 et 4, dans lequel les différentes durées d'éclairage d'une même séquence d'éclairage se suivent sans durée d'extinction entre deux durées d'éclairage.

6. Procédé selon la revendication 5 dans lequel, pour la réalisation d'images en réflexion, la zone éclairée de surface extérieure de l'objet par au moins une source lumineuse est l'intégralité d'une face visible de la surface extérieure de l'objet par le rayonnement lumineux de cette source lumineuse, et en ce qu'au moins une caméra multispectrale est agencée par rapport cette source lumineuse pour réaliser des images en réflexion de l'intégralité de cette face visible.

7. Procédé selon l'une des revendications 1 à 6 dans lequel la pluralité d'images réalisée par une même caméra multispectrale comporte :
- une image réalisée en réflexion alors que l'objet est éclairé par de la lumière blanche,
- une image réalisée en réflexion alors que l'objet est éclairé dans un domaine infrarouge d'éclairage à une longueur d'onde comprise entre 720 et 780 nm,
- une image réalisée en réflexion alors que l'objet est éclairé dans un domaine infrarouge d'éclairage à une longueur d'onde comprise entre 800 nm et 850 nm,
- une image réalisée en réflexion alors que l'objet est éclairé dans un domaine infrarouge d'éclairage à une longueur d'onde comprise entre 900 nm et 1000 nm,
- une image réalisée en réflexion alors que l'objet est éclairé dans un domaine ultraviolet d'éclairage à une longueur d'onde comprise entre 250 nm et 380 nm.

8. Dispositif d'analyse optique d'objets appartenant au groupe des fruits et légumes comprenant des moyens pour réaliser des images des objets dans différents domaines de longueur d'onde et des moyens de traitement d'image pour traiter lesdites images en vue du tri automatique desdits objets,
comportant :
- un dispositif d'éclairage comprenant une pluralité de sources lumineuses formées de diodes électroluminescentes (LED1, LED2, LED3, LED4, LED5) agencées pour pouvoir appliquer chacune un rayonnement lumineux sur au moins une portion de surface extérieure d'au moins un objet, dit objet éclairé, les différentes sources lumineuses étant adaptées pour pouvoir appliquer sélectivement sur chaque objet éclairé des rayonnements lumineux dans différents domaines de longueur d'onde d'éclairage,
- le dispositif d'éclairage étant adapté pour pouvoir appliquer un rayonnement lumineux issu d'au moins une source lumineuse sur l'intégralité d'une face visible de la surface extérieure de chaque objet éclairé par cette source lumineuse,
- un dispositif de commande (10, 20, 43a, 43b) adapté pour pouvoir commander ces sources lumineuses selon une séquence d'éclairage prédéterminée de chaque objet éclairé, successivement selon lesdits différents domaines de longueur d'onde d'éclairage, alors que ledit objet éclairé (6) est entraîné en rotation sur lui-même et en déplacement par un convoyeur au cours de ladite séquence d'éclairage,
- au moins une caméra couleur sensible aux rayonnements lumineux dans le domaine visible et de longueur d'onde comprise entre 380 nm et 700 nm, et aux rayonnements lumineux dans le domaine des infrarouges et de longueur d'onde comprise entre 700 nm et 1000 nm, dite caméra multispectrale (4, 4a, 4b), ayant des groupes d'éléments photosensibles aptes à détecter chacun l'une des couleurs primaires et étant orientée vers une portion de surface extérieure d'au moins un objet éclairé correspondant à toute la face visible de la surface extérieure de l'objet selon l'axe optique de la caméra multispectrale,
- chaque caméra (4, 4a, 4b) multispectrale étant choisie dans le groupe des caméras comprenant un capteur CMOS avec une matrice de filtres de couleurs, exemptes de filtre coupant les infrarouges ; et des caméras comprenant trois capteurs CMOS, un capteur CMOS pour chaque couleur primaire, exemptes de filtre coupant les infrarouges,
- le dispositif de commande (10, 20, 43a, 43b) étant adapté pour commander l'exposition de chaque caméra multispectrale (4, 4a, 4b) en synchronisme avec ladite séquence d'éclairage de façon à réaliser avec cette même caméra multispectrale une pluralité d'images dans différents domaines de longueur d'onde de prise de vues de ladite portion de surface extérieure d'au moins un objet éclairé, dont au moins une image dans un domaine visible et au moins une image dans un domaine infrarouge, avec un ajustement de la balance des blancs en fonction de la sensibilité pour chaque couleur dans ledit domaine infrarouge, de chaque groupe d'éléments photosensibles de la caméra multispectrale (4, 4a, 4b) dans ledit domaine infrarouge,
- chaque caméra multispectrale (4, 4a, 4b) comprenant une mémoire tampon de mémorisation des images, adaptée pour enregistrer ladite pluralité d'images de ladite portion de surface extérieure réalisées par une même caméra multispectrale à la vitesse maximale d'acquisition d'images par ladite caméra multispectrale, avant leur transmission aux moyens de traitement d'images.

9. Dispositif selon la revendication 8, dans lequel les sources lumineuses sont montées dans une chambre (2) optique ayant une surface interne réfléchissante dont la forme est choisie en fonction de la position des sources lumineuses de façon à permettre un éclairage uniforme des objets se trouvant dans le champ optique desdites au moins une caméra multispectrale.

10. Dispositif selon l'une quelconque des revendications 8 et 9, dans lequel chaque caméra multispectrale comprend un séquenceur programmable (43a,43b), ledit séquenceur programmable étant commandé par le dispositif de commande pour déclencher successivement chaque prise de vue et maintenir l'exposition de la caméra multispectrale pendant une durée correspondant à la capture d'une image déterminée.

11. Dispositif selon l'une des revendications 8 ou 9 dans lequel, pour la réalisation de chaque image dans un domaine infrarouge, ledit dispositif (10, 20) de commande est adapté pour ajuster la balance des blancs en fonction de la sensibilité de chaque couleur de la caméra multispectrale dans ledit domaine infrarouge.

12. Dispositif selon l'une des revendications 8 à 11 dans lequel chaque caméra (4, 4a, 4b) multispectrale est une caméra couleur comprenant un capteur CMOS et une matrice de filtres de couleurs, exempte de filtre coupant les infrarouges, et dotée d'une mémoire (42a, 42b) tampon de mémorisation des images.

13. Dispositif selon l'une des revendications 8 à 12 dans lequel les sources lumineuses (LED1, LED2, LED3, LED4, LED5) comportent au moins une LED d'éclairage en lumière blanche visible, au moins une LED d'éclairage par un rayonnement lumineux ultraviolet, et au moins une LED d'éclairage en infrarouge.

14. Dispositif de tri automatique d'objets appartenant au groupe des fruits et légumes selon des critères de tri prédéterminés comprenant :
- au moins une ligne (8) de convoyage apte à transporter les objets en regard de postes d'analyse des objets selon lesdits critères de tri, dont au moins un poste d'analyse optique,
- un automate (10) relié aux postes d'analyse pour en recevoir des signaux d'analyse,
- des postes de déchargement des objets dans une pluralité de zones de déchargement,
l'automate étant programmé pour commander le déchargement sélectif de chaque objet dans une zone de déchargement sélectionnée selon les signaux d'analyse reçus par cet automate pour cet objet,
**caractérisé en ce qu'**il comporte au moins un poste d'analyse optique formé d'un dispositif (3) d'analyse optique selon l'une des revendications 8 à13.

15. Dispositif selon la revendication 14 dans lequel chaque poste d'analyse optique comporte moins de quatre caméras (4a, 4b) multispectrales par ligne de convoyage.

16. Dispositif selon l'une des revendications 14 ou 15 comportant un unique poste (3) d'analyse optique.

17. Programme d'ordinateur comprenant une séquence d'instructions qui, lorsqu'elle est exécutée par un ordinateur, est adaptée pour mettre en œuvre toutes les étapes d'un procédé d'analyse optique d'objets selon l'une quelconque des revendications 1 à 7.

## Patentansprüche

1. Verfahren zur optischen Analyse von Objekten, die zu der Gruppe von Obst und Gemüse gehören, wobei repräsentative Bilder der Objekte in verschiedenen Aufnahmewellenlängenbereichen erstellt und für die automatische Sortierung der Objekte verarbeitet werden,
wobei:
- eine Vielzahl von Lichtquellen, die aus Leuchtdioden (LED1, LED2, LED3, LED4, LED5) gebildet sind, angeordnet sind, um jeweils eine Lichtstrahlung auf mindestens einen Abschnitt der äußeren Oberfläche mindestens eines Objekts, bezeichnet als beleuchtetes Objekt (6), anwenden zu können, wobei die verschiedenen Lichtquellen angepasst sind, um auf jedes beleuchtete Objekt selektiv Lichtstrahlung in verschiedenen Beleuchtungswellenlängenbereichen anwenden zu können,
- wobei die Lichtstrahlung aus mindestens einer Lichtquelle auf die gesamte sichtbare Seite einer äußeren Oberfläche von jedem Objekt, das von dieser Lichtquelle beleuchtet wird, angewendet wird,
- die Lichtquellen gemäß einer vorbestimmten Beleuchtungssequenz von jedem beleuchteten Objekt nacheinander gemäß den verschiedenen Beleuchtungswellenlängenbereichen gesteuert werden, wobei jedes beleuchtete Objekt (6) im Verlauf der Beleuchtungssequenz durch einen Förderer in Drehung um sich selbst versetzt und verschoben wird,
- Bilder durch mindestens eine Farbkamera (4, 4a, 4b) erstellt werden, die für Lichtstrahlung im sichtbaren Bereich und mit einer Wellenlänge zwischen 380 nm und 700 nm und für Lichtstrahlung im Infrarotbereich und mit einer Wellenlänge zwischen 700 nm und 1000 nm empfindlich ist, bezeichnet als Multispektralkamera (4, 4a, 4b), die Gruppen von lichtempfindlichen Elementen aufweist, die geeignet sind, um jeweils eine der Primärfarben zu erfassen, auf einen Abschnitt der äußeren Oberfläche mindestens eines beleuchteten Objekts gerichtet ist, der der gesamten sichtbaren Seite der äußeren Oberfläche des Objekts gemäß der optischen Achse der Multispektralkamera entspricht, und deren Belichtung synchronisiert mit der Beleuchtungssequenz gesteuert wird, um mit derselben Multispektralkamera eine Vielzahl von Bildern in verschiedenen Aufnahmewellenlängenbereichen des Abschnitts der äußeren Oberfläche mindestens eines beleuchteten Objekts (6) zu erstellen, davon mindestens ein Bild in einem sichtbaren Bereich und mindestens ein Bild in einem Infrarotbereich,
- wobei jede Multispektralkamera (4, 4a, 4b) ausgewählt ist aus der Gruppe von Kameras, umfassend einen CMOS-Sensor mit einer Matrix von Farbfiltern, die frei von Infrarot-Sperrfiltern sind; und Kameras, umfassend drei CMOS-Sensoren, einen CMOS-Sensor für jede Primärfarbe, die frei von Infrarot-Sperrfiltern sind, und
- jedes Bild in einem Infrarotbereich mit einer Anpassung des Weißabgleichs abhängig von der Empfindlichkeit für jede Farbe in dem Infrarotbereich von jeder Gruppe von lichtempfindlichen Elementen der Multispektralkamera (4, 4a, 4b) in dem Infrarotbereich erstellt wird,
- wobei jede Multispektralkamera (4, 4a, 4b) mit einem Pufferspeicher zum Speichern von Bildern versehen ist, die Vielzahl von Bildern des äußeren Oberflächenabschnitts von einer einzigen Multispektralkamera mit der maximalen Bilderfassungsgeschwindigkeit durch die Multispektralkamera erstellt und in dem Pufferspeicher (42a, 42b) der Multispektralkamera gespeichert werden.

2. Verfahren nach Anspruch 1, wobei die Vielzahl von Bildern des äußeren Oberflächenabschnitts, die von ein und derselben Multispektralkamera (4, 4a, 4b) erstellt werden, die zwischen zwei und zehn - insbesondere drei bis fünf - aufeinanderfolgende Bilder desselben beleuchteten Objekts in verschiedenen Wellenlängenbereichen umfasst, davon mindestens ein Bild in einem sichtbaren Bereich und mindestens ein Bild in einem Infrarotbereich, wobei die Vielzahl von Bildern während einer Beleuchtungssequenz mit einer Gesamtdauer der Beleuchtungssequenz von weniger als 10 ms, insbesondere zwischen 1 ms und 5 ms, erstellt wird.

3. Verfahren nach einem der Ansprüche 1 und 2, wobei die Beleuchtungssequenz aus einer Abfolge von Beleuchtungsdauern gebildet wird, wobei ein Teil der Lichtquellen (LED1, LED2, LED3, LED4, LED5) während jeder Beleuchtungsdauer aktiviert wird, wobei dieser Teil der Lichtquellen gewählt wird, um jedes Objekt in einem der Beleuchtungswellenlängenbereiche zu beleuchten, dass jede Beleuchtungsdauer zwischen 0,1 ms und 5 ms liegt, und dass die Belichtung einer Multispektralkamera (4, 4a, 4b) mit einer Verzögerung in Bezug auf die Aktivierung einer Beleuchtungsdauer ausgelöst wird, wobei diese Verzögerung gewählt ist, damit jede Lichtquelle des Teils der Lichtquellen (LED1, LED2, LED3, LED4, LED5) dieser Beleuchtungsdauer tatsächlich vollständig eingeschaltet und aktiv ist, bevor die Multispektralkamera ausgelöst wird.

4. Verfahren nach Anspruch 3, wobei sich die Beleuchtungswellenlängenbereiche von zwei aufeinanderfolgenden Beleuchtungsdauern die Beleuchtungssequenz voneinander unterscheiden.

5. Verfahren nach einem der Ansprüche 3 oder 4, wobei die verschiedenen Beleuchtungsdauern einer Beleuchtungssequenz ohne Ausschaltdauer zwischen zwei Beleuchtungsdauern aufeinander folgen.

6. Verfahren nach Anspruch 5, wobei der beleuchtete Bereich der äußeren Oberfläche des Objekts für die Erstellung von Reflexionsbildern durch mindestens eine Lichtquelle die Gesamtheit einer durch die Lichtstrahlung dieser Lichtquelle sichtbaren Seite der äußeren Oberfläche des Objekts ist, und dass mindestens eine Multispektralkamera in Bezug auf diese Lichtquelle angeordnet ist, um Reflexionsbilder der Gesamtheit dieser sichtbaren Seite zu erstellen.

7. Verfahren nach einem der Ansprüche 1 bis 6, wobei die Vielzahl von Bildern, die von einer einzelnen Multispektralkamera erstellt werden, Folgendes umfasst:
- ein Bild, das als Reflexion erstellt wird, während das Objekt durch das weiße Licht beleuchtet wird,
- ein Bild, das als Reflexion erstellt wird, während das Objekt in einem Infrarot-Beleuchtungsbereich mit einer Wellenlänge zwischen 720 und 780 nm beleuchtet wird,
- ein Bild, das als Reflexion erstellt wird, während das Objekt in einem Infrarot-Beleuchtungsbereich mit einer Wellenlänge zwischen 800 und 850 nm beleuchtet wird,
- ein Bild, das als Reflexion erstellt wird, während das Objekt in einem Infrarot-Beleuchtungsbereich mit einer Wellenlänge zwischen 900 und 1000 nm beleuchtet wird,
- ein Bild, das als Reflexion erstellt wird, während das Objekt in einem Ultraviolett-Beleuchtungsbereich mit einer Wellenlänge zwischen 250 und 380 nm beleuchtet wird.

8. Vorrichtung zur optischen Analyse von Objekten, die zu der Gruppe von Obst und Gemüse gehören, umfassend Einrichtungen zum Erstellen von Bildern der Objekte in verschiedenen Wellenlängenbereichen und Bildverarbeitungseinrichtungen zum Verarbeiten der Bilder für die automatische Sortierung der Objekte,
umfassend:
- eine Beleuchtungsvorrichtung, umfassend eine Vielzahl von Lichtquellen, die aus Leuchtdioden (LED1, LED2, LED3, LED4, LED5) gebildet sind, die angeordnet sind, um jeweils eine Lichtstrahlung auf mindestens einen Abschnitt der äußeren Oberfläche mindestens eines Objekts, bezeichnet als beleuchtetes Objekt, anwenden zu können, wobei die verschiedenen Lichtquellen angepasst sind, um auf jedes beleuchtete Objekt selektiv Lichtstrahlung in verschiedenen Beleuchtungswellenlängenbereichen anwenden zu können,
- wobei die Beleuchtungsvorrichtung angepasst ist, um eine Lichtstrahlung aus mindestens einer Lichtquelle auf die gesamte sichtbare Seite einer äußeren Oberfläche von jedem Objekt, das von dieser Lichtquelle beleuchtet wird, anwenden zu können,
- eine Steuervorrichtung (10, 20, 43a, 43b), die geeignet ist, um diese Lichtquellen gemäß einer vorbestimmten Beleuchtungssequenz von jedem beleuchteten Objekt nacheinander gemäß den verschiedenen Beleuchtungswellenlängenbereichen steuern zu können, während das beleuchtete Objekt (6) im Verlauf der Beleuchtungssequenz durch einen Förderer in Drehung um sich selbst versetzt und verschoben wird,
- mindestens eine Farbkamera, die für Lichtstrahlung, die für Lichtstrahlung im sichtbaren Bereich und mit einer Wellenlänge zwischen 380 nm und 700 nm und für Lichtstrahlung im Infrarotbereich und mit einer Wellenlänge zwischen 700 nm und 1000 nm empfindlich ist, bezeichnet als Multispektralkamera (4, 4a, 4b), die Gruppen von lichtempfindlichen Elementen aufweist, die geeignet sind, um jeweils eine der Primärfarben zu erfassen, und auf einen Abschnitt der äußeren Oberfläche mindestens eines beleuchteten Objekts gerichtet ist, der der gesamten sichtbaren Seite der äußeren Oberfläche des Objekts gemäß der optischen Achse der Multispektralkamera entspricht,
- wobei jede Multispektralkamera (4, 4a, 4b) ausgewählt ist aus der Gruppe von Kameras, umfassend einen CMOS-Sensor mit einer Matrix von Farbfiltern, die frei von Infrarot-Sperrfiltern sind; und Kameras, umfassend drei CMOS-Sensoren, einen CMOS-Sensor für jede Primärfarbe, die frei von Infrarot-Sperrfiltern sind,
- wobei die Steuervorrichtung (10, 20, 43a, 43b) angepasst ist, um die Belichtung jeder Multispektralkamera (4, 4a, 4b) synchronisiert mit der Beleuchtungssequenz zu steuern, um mit derselben Multispektralkamera eine Vielzahl von Bildern in verschiedenen Aufnahmewellenlängenbereichen des Teils der äußeren Oberfläche mindestens eines beleuchteten Objekts zu erstellen, davon mindestens ein Bild in einem sichtbaren Bereich und mindestens ein Bild in einem Infrarotbereich, mit einer Anpassung des Weißabgleichs gemäß der Empfindlichkeit für jede Farbe in dem Infrarotbereich von jeder Gruppe von lichtempfindlichen Elementen der Multispektralkamera (4, 4a, 4b) in dem Infrarotbereich,
- jede Multispektralkamera (4, 4a, 4b) umfassend einen Pufferspeicher zum Speichern von Bildern, der angepasst ist, um die Vielzahl von Bildern des äußeren Oberflächenabschnitts, die von einer einzelnen Multispektralkamera mit der maximalen Bilderfassungsgeschwindigkeit der Multispektralkamera erstellt werden, zu speichern, bevor sie an die Bildverarbeitungseinrichtungen übertragen werden.

9. Vorrichtung nach Anspruch 8, wobei die Lichtquellen in einer optischen Kammer (2) montiert sind, die eine reflektierende innere Oberfläche aufweist, deren Form abhängig von der Position der Lichtquellen gewählt ist, um eine gleichmäßige Beleuchtung von Objekten zu ermöglichen, die sich in dem optischen Feld der mindestens einen Multispektralkamera befinden.

10. Vorrichtung nach einem der Ansprüche 8 oder 9, wobei jede Multispektralkamera einen programmierbaren Sequenzer (43a, 43b) umfasst, wobei der programmierbare Sequenzer von der Steuervorrichtung gesteuert wird, um nacheinander jede Bildaufnahme auszulösen und die Belichtung der Multispektralkamera über eine Dauer aufrechtzuerhalten, die der Aufnahme eines bestimmten Bilds entspricht.

11. Vorrichtung nach einem der Ansprüche 8 oder 9, wobei die Steuervorrichtung (10, 20) für die Erstellung von jedem Bild in einem Infrarotbereich angepasst ist, um den Weißabgleich abhängig von der Empfindlichkeit jeder Farbe der Multispektralkamera in dem Infrarotbereich einzustellen.

12. Vorrichtung nach einem der Ansprüche 8 bis 11, wobei jede Multispektralkamera (4, 4a, 4b) eine Farbkamera ist, umfassend einen CMOS-Sensor und eine Matrix von Farbfiltern, die frei von Infrarot-Sperrfiltern ist und mit einem Pufferspeicher (42a, 42b) zum Speichern von Bildern ausgestattet ist.

13. Vorrichtung nach einem der Ansprüche 8 bis 12, wobei die Lichtquellen (LED1, LED2, LED3, LED4, LED5) mindestens eine LED zum Beleuchten mit sichtbarem Weißlicht, mindestens eine LED zum Beleuchten mit ultravioletter Lichtstrahlung und mindestens eine LED zum Beleuchten mit Infrarotlicht umfassen.

14. Vorrichtung zum automatischen Sortieren von Objekten, die zu der Gruppe der Obst und Gemüse gehören, gemäß vorbestimmten Sortierkriterien, umfassend:
- mindestens eine Förderlinie (8), die geeignet ist, um die Objekte vor Analysestationen der Objekte gemäß den Sortierkriterien zu transportieren, davon mindestens eine optische Analysestation,
- einen Automaten (10), der mit den Analysestationen verbunden ist, um Analysesignale davon zu empfangen,
- Entladestationen der Objekte in einer Vielzahl von Entladezonen,
wobei der Automat programmiert ist, um das selektive Entladen von jedem Objekt in eine Entladezone zu steuern, die gemäß den Analysesignalen ausgewählt wird, die von diesem Automaten für dieses Objekt empfangen werden,
**dadurch gekennzeichnet, dass** sie mindestens eine optische Analysestation umfasst, die aus einer Vorrichtung (3) zur optischen Analyse nach einem der Ansprüche 8 bis 13 gebildet ist.

15. Vorrichtung nach Anspruch 14, wobei jede optische Analysestation weniger als vier Multispektralkameras (4a, 4b) pro Förderlinie umfasst.

16. Vorrichtung nach einem der Ansprüche 14 oder 15 umfassend eine einzige Station (3) zur optischen Analyse.

17. Computerprogramm, umfassend ein e Anweisungssequenz, die, wenn sie von einem Computer ausgeführt wird, angepasst ist, um alle Schritte eines Verfahrens zur optischen Analyse von Objekten nach einem der Ansprüche 1 bis 7 durchzuführen.

## Claims

1. A method of optically analyzing objects belonging to the fruit and vegetable group in which images representative of the objects are produced in different imaging wavelength ranges and are processed for automatic sorting of the objects,
wherein:
- a plurality of light sources formed by light-emitting diodes (LED1, LED2, LED3, LED4, LED5) are arranged to be able each to apply light radiation to at least one external surface portion of at least one object, called illuminated object (6), the different light sources being adapted to be able to apply light radiation in different illumination wavelength ranges selectively to each illuminated object,
- the light radiation from at least one light source is applied to the whole of a visible face of the external surface of each object illuminated by this light source,
- the light sources are controlled according to a predetermined illumination sequence for each illuminated object in succession according to said different illumination wavelength ranges, each illuminated object (6) being rotated around itself and moved by a conveyor during said illumination sequence,
- images are produced by at least one color camera (4, 4a, 4b) sensitive to light radiation in the visible range and with a wavelength of between 380 nm and 700 nm and to light radiation in the infrared range and with a wavelength of between 700 nm and 1000 nm, called multispectral camera (4, 4a, 4b), having groups of photosensitive elements each able to detect one of the primary colors, being orientated toward an external surface portion of at least one illuminated object corresponding to the whole visible face of the external surface of the object on the optical axis of the multispectral camera, and the exposure of which is controlled in synchronism with said illumination sequence so as to produce, with this same multispectral camera, a plurality of images in different imaging wavelength ranges of said external surface portion of at least one illuminated object (6), including at least one image in a visible range and at least one image in an infrared range,
- each multispectral camera (4, 4a, 4b) is chosen from the group of cameras comprising a CMOS sensor with a matrix of color filters without an infrared cut-off filter; and cameras comprising three CMOS sensors, one CMOS sensor for each primary color, without an infrared cut-off filter, and
- each image in an infrared range is produced with an adjustment of the white balance according to the sensitivity for each color of each group of photosensitive elements of the multispectral camera (4, 4a, 4b) in said infrared range,
- each multispectral camera (4, 4a, 4b) being provided with a buffer memory for storing images, said plurality of images of said external surface portion is produced by the same multispectral camera at the maximum image acquisition speed by said multispectral camera, and is stored in the buffer memory (42a, 42b) of said multispectral camera.

2. The method according to claim 1, wherein the plurality of images of said external surface portion produced by a same multispectral camera (4, 4a, 4b) comprises between two and ten-in particular from three to five-successive images of the same illuminated object, in different wavelength ranges, including at least one image in a visible range and at least one image in an infrared range, said plurality of images being produced during an illumination sequence in a total duration of the illumination sequence less than 10 ms, in particular between 1 ms and 5 ms.

3. The method according to any one of claims 1 and 2, wherein said illumination sequence is formed of a succession of illumination periods, some of said light sources (LED1, LED2, LED3, LED4, LED5) being activated during each illumination period, this part of said light sources being chosen to illuminate each object in one of said illumination wavelength ranges, in that each illumination period is between 0.1 ms and 5 ms, and in that the exposure of a multispectral camera (4, 4a, 4b) is triggered with a delay with respect to the activation of an illumination period, this delay being chosen so that each light source of said part of said light sources (LED1, LED2, LED3, LED4, LED5) of this illumination period is effectively completely switched on and active before the multispectral camera is triggered.

4. The method according to claim 3, wherein the illumination wavelength ranges of two successive illumination periods of the illumination sequence are distinct from one another.

5. The method according to any one of claims 3 and 4, wherein the different illumination periods of a same illumination sequence follow one another with no extinction period between two illumination periods.

6. The method according to claim 5, wherein, to produce images by reflection, the external surface zone of the object illuminated by at least one light source is the whole of a face of the external surface of the object visible by the light radiation of this light source, and in that at least one multispectral camera is arranged relative to this light source to produce images by reflection of the whole of this visible face.

7. The method according to one of claims 1 to 6, wherein the plurality of images produced by a same multispectral camera comprises:
- an image produced by reflection while the object is illuminated by white light,
- an image produced by reflection while the object is illuminated in an infrared illumination range at a wavelength between 720 and 780 nm,
- an image produced by reflection while the object is illuminated in an infrared illumination range at a wavelength between 800 nm and 850 nm,
- an image produced by reflection while the object is illuminated in an infrared illumination range at a wavelength between 900 nm and 1000 nm,
- an image produced by reflection while the object is illuminated in an ultraviolet illumination range at a wavelength between 250 nm and 380 nm.

8. A device for optically analyzing objects belonging to the fruits and vegetables group, comprising means for producing images of the objects in different wavelength ranges and image processing means for processing said images for automatic sorting of said objects, comprising:
- an illumination device comprising a plurality of light sources formed by light-emitting diodes (LED1, LED2, LED3, LED4, LED5) arranged to be able each to apply light radiation to at least an external surface portion of at least one object, called illuminated object, the different light sources being adapted to be able to apply light radiation in different illumination wavelength ranges selectively to each illuminated object,
- the illumination device being adapted to be able to apply light radiation from at least one light source to the whole of a visible face of the external surface of each object illuminated by this light source,
- a control device (10, 20, 43a, 43b) adapted to be able to control these light sources according to a predetermined sequence of illumination of each illuminated object in succession according to the different illumination wavelength ranges, whereas said illuminated object (6) is rotated around itself and moved by a conveyor during said illumination sequence,
- at least one color camera sensitive to light radiation in the visible range and with a wavelength of between 380 nm and 700 nm, and to light radiation in the infrared range and with a wavelength of between 700 nm and 1000 nm, called multispectral camera (4, 4a, 4b), having groups of photosensitive elements each able to detect one of the primary colors and being oriented toward an external surface portion of at least one illuminated object corresponding to the whole visible face of the external surface of the object on the optical axis of the multispectral camera,
- each multispectral camera (4, 4a, 4b) being chosen from the group of cameras comprising a CMOS sensor with a matrix of color filters without an infrared cut-off filter; and cameras comprising three CMOS sensors, one CMOS sensor for each primary color, without an infrared cut-off filter,
- the control device (10, 20, 43a, 43b) being adapted to control the exposure of each multispectral camera (4, 4a, 4b) in synchronism with said illumination sequence so as to produce, with this same multispectral camera, a plurality of images in different imaging wavelength ranges of said external surface portion of at least one illuminated object, including at least one image in a visible range and at least one image in an infrared range, with an adjustment of the white balance based on the sensitivity for each color in said infrared range, of each group of photosensitive elements of the multispectral camera (4, 4a, 4b) in said infrared range,
- each multispectral camera (4, 4a, 4b) comprising a buffer memory for storing images, able to record said plurality of images of said external surface portion produced by a same multispectral camera at the maximum image acquisition speed by said multispectral camera, before they are transmitted to the image processing means.

9. The device according to claim 8, wherein the light sources are mounted in an optical chamber (2) having a reflective internal surface whose shape is chosen depending on the position of the light sources so as to permit uniform illumination of the objects in the optical field of said at least one multispectral camera.

10. The device according to any one of claims 8 and 9, wherein each multispectral camera comprises a programmable sequencer (43a, 43b), said programmable sequencer being controlled by the control device to successively trigger each acquisition and to maintain the exposure of the multispectral camera for a period corresponding to the capture of a determined image.

11. The device according to one of claims 8 or 9, wherein, to produce each image in an infrared range, said control device (10, 20) is able to adjust the white balance based on the sensitivity of each color of the multispectral camera in said infrared range.

12. The device according to one of claims 8 to 11, wherein each multispectral camera (4, 4a, 4b) is a color camera comprising a CMOS sensor and a color filter matrix, without an infrared cut-off filter, and fitted with a buffer memory (42a, 42b) for storing the images.

13. The device according to one of claims 8 to 12, wherein the light sources (LED1, LED2, LED3, LED4, LED5) comprise at least one visible white light illumination LED, at least one ultraviolet light radiation illumination LED and at least one infrared illumination LED.

14. A device for automatically sorting objects belonging to the fruit and vegetable group according to predetermined sorting criteria, comprising:
- at least one conveying line (8) able to transport the objects in front of stations for analyzing the objects in accordance with said sorting criteria, including at least one optical analysis station,
- an automated machine (10) connected to the analysis stations to receive analysis signals therefrom,
- stations for discharging the objects in a plurality of discharging regions,
the automated machine being programmed to control the selective discharging of each object in a discharging region selected in accordance with the analysis signals received by this automated machine for this object,
**characterized in that** it comprises at least one optical analysis station formed by an optical analysis device (3) according to one of claims 8 to 13.

15. The device according to claim 14, wherein each optical analysis station comprises less than four multispectral cameras (4a, 4b) per conveying line.

16. The device according to one of claims 14 or 15, comprising a single optical analysis station (3).

17. A computer program comprising a sequence of instructions which, when it is executed by a computer, is able to implement all the steps of a method for optically analyzing objects according to any one of claims 1 to 7.
